# EUROPEAN PATENT SPECIFICATION

(11) **EP 3 878 845 B1**
(45) Date of publication and mention of the grant of the patent: **21.06.2023**
(21) Application number: 19882457.5
(22) Date of filing: 06.11.2019
(51) Int. Cl.: C07D 317/42

(54) **PERFLUORO(2-METHYLENE-4-METHYL-1,3-DIOXOLANE) PRODUCTION METHOD**
PERFLUOR-(2-METHYLEN-4-METHYL-1,3-DIOXOLAN)-HERSTELLUNGSVERFAHREN
PROCÉDÉ DE FABRICATION DE PERFLUORO (2-MÉTHYLÈN-4-MÉTHYL-1,3-DIOXOLANE)

(30) Priority: 06.11.2018 JP 2018208937; 06.11.2018 JP 2018208938
(43) Date of publication of application: 15.09.2021
(73) Proprietor: Tosoh Corporation, Yamaguchi 746-8501 (JP); Tosoh Finechem Corporation, Shunan-shi, Yamaguchi 746-0006 (JP)
(72) Inventor: MIYAUCHI Hideki, Shunan-shi, Yamaguchi 746-0006 (JP); MATSUO Hiroshi, Shunan-shi, Yamaguchi 746-0006 (JP); MIYASHITA Yuichi, Shunan-shi, Yamaguchi 746-0006 (JP); FUJI Akihiro, Shunan-shi, Yamaguchi 746-0006 (JP); SHIGETA Yusuke, Shunan-shi, Yamaguchi 746-0006 (JP); NISHIDA Shota, Shunan-shi, Yamaguchi 746-0006 (JP); KONDO Norihisa, Shunan-shi, Yamaguchi 746-0006 (JP); WATANABE Makoto, Yokkaichi-shi, Mie 510-8540 (JP)
(74) Representative: Vigand, Philippe
(86) International application number: PCT/JP2019/043350
(87) International publication number: WO 2020/095915

(56) References cited:
- JP-A- 2007 504 125
- Franti ek Mike et al: "Synthesis and Characterization of an Amorphous Perfluoropolymer: Poly(perfluoro-2-methylene-4-methyl-1,3-di oxolane)", Macromolecules, vol. 38, no. 10, 2005, pages 4237-4245, XP055702867, DOI: 10.1021/ma050085u

## Description

### [Technical Field]

The present invention relates to a method of producing perfluoro(2-methylene-4-methyl-1,3-dioxolane).

### [Background Art]

Perfluoro(2-methylene-4-methyl-1,3-dioxolane) is a compound used as a synthetic raw material for poly[(perfluoro(2-methylene-4-methyl-1,3-dioxolane)], which is prospective as a resin for a gas separation membrane, a transparent resin for optical fibers, or the like.

As a method of producing perfluoro(2-methylene-4-methyl-1,3-dioxolane), a method in which perfluoro(2,4-dimethyl-2-fluoroformyl-1,3-dioxolane) is caused to flow in a gas state on a sodium carbonate solid catalyst (Patent Literature 1), a method in which 2-methoxycarbonyl-2-trifluoromethyl-4-methyl-1,3-dioxolane is fluorinated with fluorine gas, and an ester is hydrolyzed with a potassium hydroxide aqueous solution to form a potassium salt and dried as a solid, and then thermally decomposed (Patent Literature 2), and a method in which a mixture of potassium perfluoro(2,4-dimethyl-1,3-dioxolane-2-yl)carboxylate and potassium fluoride is isolated as a solid, dried and then mixed with a bis(2-ethoxyethyl)ether solvent, and thermally decomposed (Non Patent Literature 1) are known.

### [Citation List]

### [Patent Literature]

[PTL 1] U.S. Patent No. 3308107
[PTL 2] Japanese Patent No. 4776536

### [Non Patent Literature]

[NPL 1] F. Mikes, et. al., Macromolecules 2005, 38, 4237-4245

### [Summary of Invention]

In the method described in Patent Literature 1, since a reaction at a high temperature of 295°C is required and highly toxic fluorophosgenes are generated as by-products, it is difficult to perform the method on an industrial scale.

In addition, in the method described in Patent Literature 2, since a reaction at a high temperature of 250°C to 280°C is required, and a decarboxylation reaction is caused in a solid phase system, it is difficult to perform the method on an industrial scale.

On the other hand, in the method described in Non Patent Literature 1, a decarboxylation reaction is a liquid phase system and the reaction temperature is relatively low at 130°C. However, it has problems that a target product which is perfluoro(2-methylene-4-methyl-1,3-dioxolane) is obtained as a mixture with 2-hydro-perfluoro(2,4-dimethyl-1,3-dioxolane) as a by-product, and it is very difficult to perform separation between the target product and the by-product by distillation.

In view of the above circumstances, one aspect of the present invention provides for a method of producing perfluoro(2-methylene-4-methyl-1,3-dioxolane), which is easily performed industrially, has a high yield, and in which a by-product that is difficult to separate off is barely to be produced.

### [Solution to Problem]

The inventors conducted extensive studies regarding a method of producing perfluoro(2-methylene-4-methyl-1,3-dioxolane) and, as a result, newly found that, with at least one of the raw material perfluoro(2,4-dimethyl-2-fluoroformyl-1,3-dioxolane) and a hydrolysis product thereof being reacted with a basic aqueous solution containing one or more cations selected from the group consisting of alkali metal ions and alkaline earth metal ions, a liquid containing the produced perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts are then separated by a liquid separation operation, one or more water content reduction treatments selected from the group consisting of water evaporation and water adsorption are then performed to be, after that, used in a decarboxylation reaction in a liquid phase system, whereby perfluoro(2-methylene-4-methyl-1,3-dioxolane) can be obtained with a high yield while suppressing production of 2-hydro-perfluoro(2,4-dimethyl-1,3-dioxolane), which is difficult to separate off. In addition, a decarboxylation reaction in a liquid phase system can be easily performed on an industrial scale.

That is, one aspect of the present invention (hereinafter referred to as an "aspect A") is as follows.
[1] A method of producing perfluoro(2-methylene-4-methyl-1,3-dioxolane), the method including at least following processes (1) to (3):
   (1) reacting at least one of perfluoro(2,4-dimethyl-2-fluoroformyl-1,3-dioxolane) and a hydrolysis product thereof with a basic aqueous solution containing one or more cations selected from the group consisting of alkali metal ions and alkaline earth metal ions and then separating a liquid containing produced perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts by a liquid separation operation;
   (2) performing one or more water content reduction treatments selected from the group consisting of water evaporation and water adsorption on the liquid containing the obtained perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts to obtain perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts in a solution state or a solid state; and
   (3) causing a decarboxylation reaction in a liquid phase system with the obtained perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts in a solution state or a solid state to produce perfluoro(2-methylene-4-methyl-1,3-dioxolane).
[2] The method of producing perfluoro(2-methylene-4-methyl-1,3-dioxolane) according to [1], further including a following process (4) after the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts is separated by the liquid separation operation in the process (1):
   (4) performing an operation at least once in which the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts obtained in the process (1) is additionally brought into contact with water or water in which an inorganic salt is dissolved, a liquid separation operation is then performed such that the liquid containing the perfluoro(2,4-dimethyl-1 ,3-dioxolane-2-yl)carboxylic acid alkali metal salts or perfluoro(2,4-dimethyl-1,3-dioxolane-2-yl)carboxylic acid alkaline earth metal salts is again separated.
[3] The method of producing perfluoro(2-methylene-4-methyl-1,3-dioxolane) according to [1] or [2], further including adding one or more aprotic solvents C at least any time from among: before the basic aqueous solution containing one or more cations selected from the group consisting of alkali metal ions and alkaline earth metal ions is reacted; after the reaction; and after the liquid separation operation in the process (1).
[4] The method of producing perfluoro(2-methylene-4-methyl-1,3-dioxolane) according to [3], wherein the aprotic solvent C is an ether solvent.
[5] The method of producing perfluoro(2-methylene-4-methyl-1,3-dioxolane) according to [3] or [4], wherein the aprotic solvent C is a glycol diether solvent.
[6] The method of producing perfluoro(2-methylene-4-methyl-1,3-dioxolane) according to any one of [3] to [5], wherein the aprotic solvent C is diethylene glycol dimethyl ether.
[7] The method of producing perfluoro(2-methylene-4-methyl-1,3-dioxolane) according to any one of [1] to [6], wherein the water content reduction treatment in the process (2) includes water evaporation in the presence of one or more azeotropic solvents that form an azeotropic composition with water.
[8] The method of producing perfluoro(2-methylene-4-methyl-1,3-dioxolane) according to any one of [3] to [5], wherein the water content reduction treatment in the process (2) includes water evaporation in the presence of one or more azeotropic solvents which form an azeotropic composition with water and have a boiling point lower than that of the aprotic solvent C which is added in the process (1).
[9] The method of producing perfluoro(2-methylene-4-methyl-1,3-dioxolane) according to [7] or [8], wherein the azeotropic solvent is one or more solvents selected from the group consisting of isopropyl alcohol and toluene.
[10] The method of producing perfluoro(2-methylene-4-methyl-1,3-dioxolane) according to any one of [1] to [9], wherein the solvent contained in the liquid phase system when the decarboxylation reaction is caused in the liquid phase system in the process (3) includes a glycol diether solvent.
   Another aspect (hereinafter referred to as an "aspect B") of the present invention is as follows.
[11] A method of producing perfluoro(2-methylene-4-methyl-1,3-dioxolane), the method including a reaction distillation process in which reaction distillation is performed by continuously supplying, to a reaction distiller, a raw material solution containing perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts to produce perfluoro(2-methylene-4-methyl-1,3-dioxolane).
[12] The method of producing perfluoro(2-methylene-4-methyl-1,3-dioxolane) according to [11], wherein the reaction distillation process includes continuous supply of a raw material solution of perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts dissolved in an aprotic solvent A to an aprotic solvent B heated in the reaction distiller under a reduced pressure and performing reaction while distilling off the produced perfluoro(2-methylene-4-methyl-1,3-dioxolane) to the outside of the system, and the aprotic solvent B is a solvent which is the same as or different from the aprotic solvent A and has a boiling point equal to or higher than that of the aprotic solvent A.
[13] The method of producing perfluoro(2-methylene-4-methyl-1,3-dioxolane) according to [12], wherein the aprotic solvent A includes at least one ether solvent.
[14] The method of producing perfluoro(2-methylene-4-methyl-1,3-dioxolane) according to [12] or [13], wherein the aprotic solvent A includes at least one glycol diether solvent.
[15] The method of producing perfluoro(2-methylene-4-methyl-1,3-dioxolane) according to any one of [12] to [14], wherein the aprotic solvent A includes diethylene glycol dimethyl ether.
[16] The method of producing perfluoro(2-methylene-4-methyl-1,3-dioxolane) according to any one of [12] to [15], wherein the aprotic solvent B includes at least one alkane solvent.
[17] The method of producing perfluoro(2-methylene-4-methyl-1,3-dioxolane) according to [16], wherein the aprotic solvent B includes liquid paraffin.
[18] The method of producing perfluoro(2-methylene-4-methyl-1,3-dioxolane) according to any one of [12] to [15], wherein the aprotic solvent B includes at least one glycol diether solvent.
[19] The method of producing perfluoro(2-methylene-4-methyl-1,3-dioxolane) according to [18], wherein the aprotic solvent B includes tetraethylene glycol dimethyl ether.
[20] The method of producing perfluoro(2-methylene-4-methyl-1,3-dioxolane) according to any one of [11] to [19], wherein the raw material solution contains a perfluoro(2,4-dimethyl-1,3-dioxolane-2-yl)carboxylic acid potassium salt or a perfluoro(2,4-dimethyl-1,3-dioxolane-2-yl)carboxylic acid sodium salt.
[21] The method of producing perfluoro(2-methylene-4-methyl-1,3-dioxolane) according to any one of [11] to [20], further including following processes (1) and (2):
   (1) reacting at least one of perfluoro(2,4-dimethyl-2-fluoroformyl-1,3-dioxolane) and a hydrolysis product thereof with a basic aqueous solution containing one or more cations selected from the group consisting of alkali metal ions and alkaline earth metal ions and then separating a liquid containing produced perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts by a liquid separation operation; and
   (2) performing one or more water content reduction treatments selected from the group consisting of water evaporation and water adsorption on the liquid containing the obtained perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts to obtain perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts in a solution state or a solid state.

According to one aspect of the present invention, it is possible to provide a method of producing perfluoro(2-methylene-4-methyl-1,3-dioxolane), which is easily performed industrially, has a high yield, and in which a by-product that is difficult to separate off is barely to be produced.

### [Description of Embodiments]

Hereinafter, an aspect A and an aspect B will be described in more detail. Unless otherwise specified, the following description regarding the aspect A can also be applied to the aspect B, and the following description regarding the aspect B can also be applied to the aspect A.

### [Aspect A]

The aspect A relates to a method of producing perfluoro(2-methylene-4-methyl-1,3-dioxolane) including at least the above processes (1) to (3).

Hereinafter, the above production method will be described in more detail.

### <Process (1)>

Perfluoro(2,4-dimethyl-2-fluoroformyl-1,3-dioxolane) used as a raw material in the process (1) is represented by the following Formula 1.

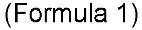

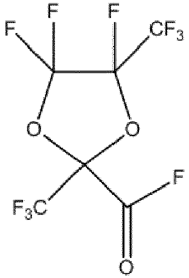

Perfluoro(2,4-dimethyl-2-fluoroformyl-1,3-dioxolane) can be obtained by a method known in literatures. For example, as described in U.S. Patent No. 3308107 or Macromolecules 2005, 38, 4237-4245, perfluoro(2,4-dimethyl-2-fluoroformyl-1,3-dioxolane) can be obtained by reacting a trifluoropyruvic acid fluoride monomer or its dimer with hexafluoropropylene oxide in the presence of cesium fluoride. In addition, the hydrolysis product of perfluoro(2,4-dimethyl-2-fluoroformyl-1,3-dioxolane) is a product produced by a reaction between perfluoro(2,4-dimethyl-2-fluoroformyl-1,3-dioxolane) and water, and is generally obtained as a mixture of perfluoro(2,4-dimethyl-1,3-dioxolane-2-yl)carboxylic acid (the following Formula 2) and hydrogen fluoride.

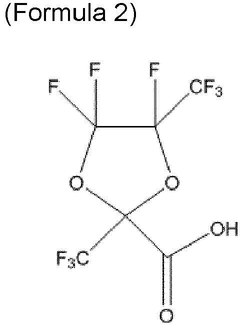

That is, in the process (1), at least one (that is, one or both) of perfluoro(2,4-dimethyl-2-fluoroformyl-1,3-dioxolane) and perfluoro(2,4-dimethyl-1,3-dioxolane-2-yl)carboxylic acid can be reacted with a basic aqueous solution containing one or more (that is, only one or two or more) cations selected from the group consisting of alkali metal ions and alkaline earth metal ions.

In the process (1), after at least one of perfluoro(2,4-dimethyl-2-fluoroformyl-1,3-dioxolane) and its hydrolysis product reacts with a basic aqueous solution containing one or more cations selected from the group consisting of alkali metal ions and alkaline earth metal ions, the liquid containing the produced perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts is separated by a liquid separation operation. The liquid separation operation can also be referred to as a layer separation operation.

Here, the perfluoro(2,4-dimethyl-1,3-dioxolane-2-yl)carboxylic acid alkali metal salt can be represented by the following Formula 3. In Formula 3, M^{l} represents an alkali metal element. The bond between O and M^{l} represents a covalent bond or an ionic bond. When representing an ionic bond, M^{l} can represent an alkali metal ion. As the perfluoro(2,4-dimethyl-1,3-dioxolane-2-yl)carboxylic acid alkali metal salts, one type of perfluoro(2,4-dimethyl-1,3-dioxolane-2-yl)carboxylic acid alkali metal salt having the same type of alkali metal element represented by M^{l} may be produced, and two or more types of perfluoro(2,4-dimethyl-1,3-dioxolane-2-yl)carboxylic acid alkali metal salts having different types of alkali metal elements represented by M^{l} may be produced.

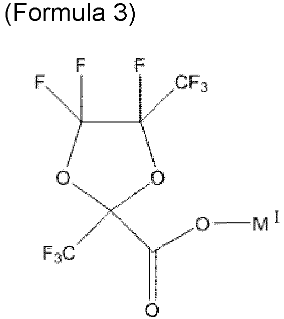

The perfluoro(2,4-dimethyl-1,3-dioxolane-2-yl)carboxylic acid alkaline earth metal salt can be represented by the following Formula 4 or Formula 5. In Formula 4 and Formula 5, each M^{l l} independently represents an alkaline earth metal element. The bond between O and M^{l l} represents a covalent bond or an ionic bond. When representing an ionic bond, M^{l l} can represent an alkaline earth metal ion. In Formula 5, the bond between M^{ll} and R represents an ionic bond. R represents a hydroxide ion (OH⁻), a hydrogen carbonate ion (HCO₃⁻), a hydrogen sulfate ion (HSO₄⁻), a hydrogen sulfite ion (HSO₃⁻), a perchlorate ion (HClO₄⁻) or a halogen ion. As the perfluoro(2,4-dimethyl-1,3-dioxolane-2-yl)carboxylic acid alkaline earth metal salts, one type of perfluoro(2,4-dimethyl-1,3-dioxolane-2-yl)carboxylic acid alkaline earth metal salt having the same type of ion represented by R may be produced, and two or more types of perfluoro(2,4-dimethyl-1,3-dioxolane-2-yl)carboxylic acid alkaline earth metal salts having different types of ions represented by R may be produced. In addition, as the perfluoro(2,4-dimethyl-1,3-dioxolane-2-yl)carboxylic acid alkaline earth metal salts, one type of perfluoro(2,4-dimethyl-1,3-dioxolane-2-yl)carboxylic acid alkaline earth metal salt having the same type of alkaline earth metal element represented by M^{ll} may be produced and two or more types of perfluoro(2,4-dimethyl-1,3-dioxolane-2-yl)carboxylic acid alkaline earth metal salts having different types of alkaline earth metal elements represented by M^{ll} may be produced.

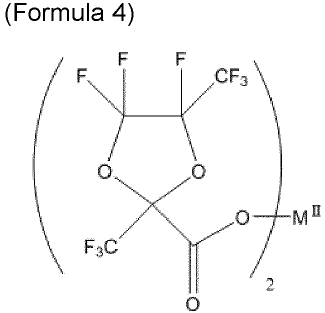

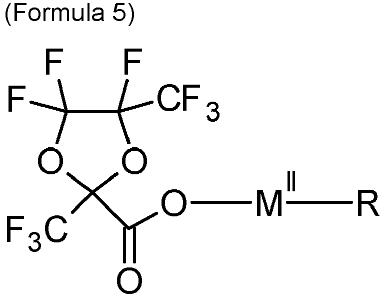

Examples of alkali metal ions include lithium ions, sodium ions, potassium ions, rubidium ions and cesium ions. In addition, examples of alkaline earth metal ions include beryllium ions, magnesium ions, calcium ions and the like. The basic aqueous solution containing one or more cations selected from the group consisting of alkali metal ions and alkaline earth metal ions can be an aqueous solution prepared by dissolving one or more salts such as hydroxides, carbonates, bicarbonates (also referred to as a "hydrogencarbonates"), and sulfites of these cations in water. The concentration of the cations in such an aqueous solution is, for example, 5 to 50 weight%, as the salt concentration. In addition, the amount of alkali metal ions used is, for example, in a molar ratio range of 0.5 to 3, with respect to perfluoro(2,4-dimethyl-2-fluoroformyl-1,3-dioxolane), and the amount of alkaline earth metal ions used is, for example, in a molar ratio range of 0.25 to 1.5, with respect to perfluoro(2,4-dimethyl-2-fluoroformyl-1,3-dioxolane).

R represents a hydroxide ion (OH⁻), a hydrogen carbonate ion (HCO₃⁻), a hydrogen sulfate ion (HSO₄⁻), a hydrogen sulfite ion (HSO₃⁻), a perchlorate ion (HClO₄⁻) or a halogen ion. Examples of halogen ions include fluoride ions (F⁻), chloride ions (F⁻), bromide ions (Br⁻), iodide ions (I⁻) and the like.

Among these, R is preferably a hydroxide ion (OH⁻) or a hydrogen carbonate ion (HCO₃⁻) in consideration of availability and reactivity of raw materials and the like.

The reaction temperature when at least one of perfluoro(2,4-dimethyl-2-fluoroformyl-1,3-dioxolane) and its hydrolysis product reacts with a basic aqueous solution containing one or more cations selected from the group consisting of alkali metal ions and alkaline earth metal ions is, for example, in a range of -10°C to +50°C, as the temperature of a reaction solution. Generally, at least one of perfluoro(2,4-dimethyl-2-fluoroformyl-1,3-dioxolane) and its hydrolysis product is sequentially added and the reaction is caused while controlling the temperature, or a basic aqueous solution containing one or more cations selected from the group consisting of alkali metal ions and alkaline earth metal ions is sequentially added and the reaction is caused while controlling the temperature. Regarding the reaction time, for example, after all raw materials are mixed, the reaction continues for 10 minutes to 1 hour, and the mixture is then left for 10 minutes to 1 hour, and a liquid separation operation can then be performed.

It tends to be difficult for the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts produced according to the above reaction to dissolve in water due to an influence of fluorine atoms even though they are salts. Therefore, the reaction solution after the reaction is generally layer-separated. A layer (organic layer) containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts, which is the target product in the process (1), is generally a lower layer. As will be described below, when an aprotic solvent C is added, the layer (organic layer) containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts, which is the target product may be the upper layer during the liquid separation operation. On the side of the aqueous layer separated from the organic layer containing the target product, the by-product alkali metal fluoride or alkaline earth metal fluoride easily dissolves. A part or all of these fluorides can be separated off by the liquid separation operation.

In addition, at at least any time before at least one of perfluoro(2,4-dimethyl-2-fluoroformyl-1,3-dioxolane) and its hydrolysis product reacts with the basic aqueous solution containing the above cations, during the reaction, after the reaction, and after the liquid separation operation to be described below, one or more aprotic solvents (referred to as an "aprotic solvent C") can be added. When the aprotic solvent C is added, the separation and removal efficiency of the by-product alkali metal fluoride or alkaline earth metal fluoride can be improved, and it is possible to reduce the loss of the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts on the side of the aqueous layer.

Regarding the aprotic solvent C, an aprotic solvent in which the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts can be dissolved is preferable, and specific examples thereof include ether solvents (ethers) such as diisopropyl ether, dibutyl ether, anisole, dimethoxyethane, diethoxyethane, diethylene glycol dimethyl ether, triethylene glycol dimethyl ether, and tetraethylene glycol dimethyl ether, esters such as ethyl acetate, butyl acetate, and ethyl propionate, ketone solvents (ketones) such as methyl ethyl ketone, cyclohexanone, and acetophenone, amide solvents (amides) such as dimethylformamide, dimethylacetamide, and N-methylpyrrolidone, nitrile solvents (nitriles) such as acetonitrile and propiononitrile, aromatic hydrocarbon solvents (aromatic hydrocarbons) such as toluene and xylene, alkane solvents (alkanes) such as heptane and octane, and mixed solvents of two or more thereof. Among these, in consideration of the solubility of the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts and the like, ether solvents are preferable, and glycol diether solvents (glycol ethers) such as diethoxyethane and diethylene glycol dimethyl ether are more preferable. The amount of the aprotic solvent C used with respect to the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts can be generally in a weight ratio range of 0.5 to 5.

In addition, after a liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts is separated by the liquid separation operation, the following process (4) can be performed.
(4) an operation is performed once or more than once, in which, in each of the operation, the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts obtained in the process (1) is brought into contact with water or water in which an inorganic salt is dissolved, and a liquid separation operation is then performed such that the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts is separated off. When the aprotic solvent C is added in the process (1), the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts can be brought into contact with water or water in which an inorganic salt is dissolved in the presence of the aprotic solvent C. On the other hand, when the aprotic solvent C is not added in the process (1), the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts can be brought into contact with water or water in which an inorganic salt is dissolved in the absence of the aprotic solvent C.

When the process (4) is performed, it is possible to further reduce the content of the alkali metal fluoride or alkaline earth metal fluoride in the organic layer. Regarding the inorganic salt here, in addition to basic salts of alkali metals or alkaline earth metal hydroxides, carbonates, bicarbonates, and sulfites, neutral salts such as chlorides, sulfates, and nitrates can be used. The process (4) is also referred to as a re-separating process. When the process (4) is performed, the process (1), the process (4) (re-separating process), and the process (2) can be performed in this order.

### <Process (2)>

In the process (2), after the process (1) or the process (4), one or more water content reduction treatments selected from the group consisting of water evaporation and water adsorption is performed on the liquid containing the obtained perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts. Therefore, it is possible to obtain perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts, in a solution state or a solid state, having a smaller water content than that before the water content reduction treatment is performed. In the process (1), it is possible to obtain a liquid having a small fluoride content as the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts. That is, it is possible to obtain a liquid having a low content of water bound to or interacted with fluoride. Therefore, by performing an operation such as water evaporation and/or removal by adsorption that is easy to perform industrially, it is possible to reduce the content of water contained in the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts.

Water evaporation is preferably evaporation by distillation off. The liquid temperature during distillation is, for example, 0°C to 100°C, and preferably 20°C to 70°C, and generally, it is efficient to perform distillation under a reduced pressure of 0.1 kPa to 50 kPa. In this case, when one or more azeotropic solvents that form an azeotropic composition with water are provided, since the azeotropic composition containing water can be evaporated at a temperature lower than the boiling point of water, it is possible to reduce the load on the device without need for a high vacuum. The azeotropic solvent is not particularly limited as long as it is a solvent that forms an azeotropic composition with water. n-propyl alcohol, isopropropyl alcohol, n-butanol, isobutyl alcohol, t-butyl alcohol, tetrahydrofuran, 1,4-dioxane, 1,2-dimethoxyethane, ethyl acetate, ethyl methyl ketone, acetonitrile, 1,2-dichloroethane, toluene, o-xylene, cyclohexane and the like have a high content of water in an azeotropic mixture and can be suitably used, and among these, at least one of isopropyl alcohol and toluene is preferable. In addition, when the aprotic solvent C is added in the process (1), the azeotropic solvent provided in the process (2) may be the same as or different from the aprotic solvent C. In this case, when the boiling point of the azeotropic solvent provided in the process (2) is lower than the boiling point of the aprotic solvent C added in the process (1), the perfluoro(2,4-dimethyl-1,3-dioxolane-2-yl)carboxylic acid alkali metal salt or perfluoro(2,4-dimethyl-1,3-dioxolane-2-yl)carboxylic acid alkaline earth metal salt obtained after evaporation according to azeotropic water is suitable because it can be easily obtained in the form of a solution containing the aprotic solvent C and can be directly used in the process (3).

In addition, regarding an adsorption material used for reducing the water content by removal by adsorption, inorganic salts that can form hydrated salts such as anhydrous sodium sulfate, anhydrous magnesium sulfate, and anhydrous calcium chloride, physical adsorption materials such as natural zeolite and synthetic zeolite and the like can be used, and these can be used in combination in stages. The amount of the adsorption material used can be generally 0.1 to 5 times the amount of water contained in the liquid containing the obtained perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts in the process (1) as a specific adsorption amount of the adsorption material. Regarding the temperature when removal by adsorption is performed, generally removal can be performed under a working environment with an atmosphere temperature of 0°C to 50°C and an atmospheric pressure, and the time for which the adsorption material is brought into contact can be generally, in a range of 10 minutes to 24 hours.

### <Process (3)>

In the process (3), a decarboxylation reaction is caused in a liquid phase system using perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts obtained in a solution state or a solid state in the process (2), and a target compound perfluoro(2-methylene-4-methyl-1,3-dioxolane) is produced.

Here, perfluoro(2-methylene-4-methyl-1,3-dioxolane) can be represented by the following Formula 6.

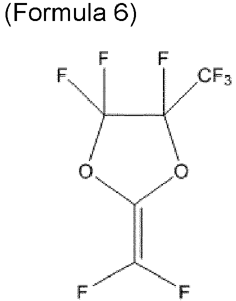

When perfluoro(2,4-dimethyl-1,3-dioxolane-2-yl)carboxylic acid alkali metal salt or perfluoro(2,4-dimethyl-1,3-dioxolane-2-yl)carboxylic acid alkaline earth metal salt is obtained in a liquid state in the process (2), the liquid can be directly used in the decarboxylation reaction, and as necessary, a solvent can be added for the reaction. In addition, when perfluoro(2,4-dimethyl-1,3-dioxolane-2-yl)carboxylic acid alkali metal salt or perfluoro(2,4-dimethyl-1,3-dioxolane-2-yl)carboxylic acid alkaline earth metal salt is obtained in a solid state in the process (2), the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts are dissolved or dispersed using a solvent and a decarboxylation reaction can be caused. Examples of solvents used here include ether solvents (ethers) such as diisopropyl ether, dibutyl ether, anisole, dimethoxyethane, diethoxyethane, diethylene glycol dimethyl ether, triethylene glycol dimethyl ether, and tetraethylene glycol dimethyl ether, ester solvents (esters) such as ethyl acetate, butyl acetate, and ethyl propionate, ketone solvents (ketones) such as ethyl methyl ketone, cyclohexanone, and acetophenone, amide solvents (amides) such as dimethylformamide, dimethylacetamide, and N-methylpyrrolidone, nitriles such as acetonitrile and propiononitrile, aromatic hydrocarbon solvents (aromatic hydrocarbons) such as toluene and xylene, alkane solvents (alkanes) such as heptane and octane, and mixed solvents of two or more thereof, and may be the same as or different from the aprotic solvent C added in the process (1) or the azeotropic solvent provided in the process (2). In consideration of the solubility of the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts and the yield of perfluoro(2-methylene-4-methyl-1,3-dioxolane), glycol diether solvents (glycol ethers) can be preferably used.

The reaction temperature when a decarboxylation reaction is performed can be, for example, in a range of 100°C to 200°C, the reaction is caused under a reduced pressure of 1 kPa to 90 kPa, and the reaction is preferably caused while distilling the produced perfluoro(2-methylene-4-methyl-1,3-dioxolane). In addition, perfluoro(2,4-dimethyl-1,3-dioxolane-2-yl)carboxylic acid alkali metal salt or perfluoro(2,4-dimethyl-1,3-dioxolane-2-yl)carboxylic acid alkaline earth metal in a solution state can be continuously added dropwise to the solvent heated in the same temperature range, and reacted while continuously distilling the produced perfluoro(2-methylene-4-methyl-1,3-dioxolane). The reaction time can be generally 0.5 hours to 24 hours. Perfluoro(2-methylene-4-methyl-1,3-dioxolane) distilled during the reaction can be collected in a trap cooled to, for example, -20°C to -80°C. In addition, when depressurization is performed using a diaphragm pump, a pump outlet gas whose pressure has been increased to atmospheric pressure or higher is cooled to the boiling point of perfluoro(2-methylene-4-methyl-1,3-dioxolane) or lower, and thus the target product perfluoro(2-methylene-4-methyl-1,3-dioxolane) can be collected.

According to the aspect A, it is possible to obtain perfluoro(2-methylene-4-methyl-1,3-dioxolane) having a low content of 2-hydro-perfluoro(2,4-dimethyl-1,3-dioxolane) as a by-product. Therefore, the obtained perfluoro(2-methylene-4-methyl-1,3-dioxolane) may be directly used as a synthetic raw material for poly[perfluoro(2-methylene-4-methyl-1,3-dioxolane)]. In addition, as necessary, purification may be performed by precision distillation.

### [Aspect B]

A method of producing perfluoro(2-methylene-4-methyl-1,3-dioxolane) according to the aspect B includes a reaction distillation process in which a raw material solution containing perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts is continuously supplied to a reaction distiller, reaction distillation is performed and perfluoro(2-methylene-4-methyl-1,3-dioxolane) is produced.

Hereinafter, the above production method will be described in more detail.

In the present invention and this specification, "reaction distillation" refers to a reaction form in which perfluoro(2-methylene-4-methyl-1,3-dioxolane) is produced and simultaneously is removed to the outside of a reaction system. The above production method is easy to perform industrially because production of perfluoro(2-methylene-4-methyl-1,3-dioxolane) according to a decarboxylation reaction of the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts is performed in a liquid phase system. The reaction distillation is preferably performed under a reduced pressure. When reaction distillation is performed under a reduced pressure, it is possible to promote distillation of perfluoro(2-methylene-4-methyl-1,3-dioxolane), and as a result, it is possible to obtain perfluoro(2-methylene-4-methyl-1,3-dioxolane) with a higher yield.

In the present invention and this specification, the phrases "continuously supply" and "continuous supply" are used to indicate a supply form other than that in which all components to be supplied are supplied at one time. For example, "continuously supply" and "continuous supply" include a supply form in which a component to be supplied is added dropwise over a predetermined time.

For the target product perfluoro(2-methylene-4-methyl-1,3-dioxolane), and perfluoro(2,4-dimethyl-1,3-dioxolane-2-yl)carboxylic acid alkali metal salts and perfluoro(2,4-dimethyl-1,3-dioxolane-2-yl)carboxylic acid alkaline earth metal salts (hereinafter referred to as a "raw metal salt") contained in the raw material solution, the previous descriptions in the aspect A can be referred to.

In addition, examples of perfluoro(2,4-dimethyl-1,3-dioxolane-2-yl)carboxylic acid alkali metal salts include lithium salts, sodium salts, potassium salts, rubidium salts and cesium salts.

Examples of perfluoro(2,4-dimethyl-1,3-dioxolane-2-yl)carboxylic acid alkaline earth metal salts include beryllium salts, magnesium salts, calcium salts and the like.

In consideration of the industrial availability and reactivity, the raw metal salt is preferably an alkali metal salt and more preferably a sodium salt and a potassium salt.

A raw metal salt can be obtained by, for example, a method known in literatures. As described in, for example, U.S. Patent No. 3308107 or Macromolecules 2005, 38, 4237-4245, a raw metal salt can be obtained by a method in which perfluoro(2,4-dimethyl-2-fluoroformyl-1,3-dioxolane) obtained by reacting a trifluoropyruvic acid fluoride monomer or its dimer with hexafluoropropylene oxide in the presence of cesium fluoride is hydrolyzed, and neutralized with an alkali metal hydroxide, an alkali metal carbonate, an alkaline earth metal hydroxide, an alkaline earth metal carbonate or the like, and a dehydration treatment such as depressurization and heating is performed. Here, the raw metal salt may contain an equimolar amount or less of alkali metal fluorides or alkaline earth metal fluorides which are by-products during hydrolysis and neutralization.

In addition, one form of the aspect B may include obtaining a raw metal salt through the process (1) and the process (2) included in the aspect A. Details of the process (1) and the process (2) are the same as those described above in the aspect A.

In the above production method, preferably, the raw metal salt dissolved in the aprotic solvent A may be reacted under a reduced pressure while adding the heated aprotic solvent B.

The aprotic solvent A is preferably an aprotic solvent in which a raw metal salt can be dissolved. Specific examples thereof include ether solvents (ethers) such as diisopropyl ether, dibutyl ether, anisole, dimethoxyethane, diethoxyethane, diethylene glycol dimethyl ether, triethylene glycol dimethyl ether, and tetraethylene glycol dimethyl ether, ester solvents (esters) such as ethyl acetate, butyl acetate, and ethyl propionate, ketone solvents (ketones) such as methyl ethyl ketone, cyclohexanone, and acetophenone, amide solvents (amides) such as dimethylformamide, dimethylacetamide, and N-methylpyrrolidone, nitrile solvents (nitriles) such as acetonitrile and propiononitrile, aromatic hydrocarbon solvents (aromatic hydrocarbons) such as toluene and xylene, alkane solvents (alkanes) such as heptane and octane, and mixed solvents of two or more thereof. Among these, in consideration of the solubility of the raw metal salt and the yield of perfluoro(2-methylene-4-methyl-1,3-dioxolane), the aprotic solvent A preferably includes at least one ether solvent and more preferably includes glycol diether solvent (glycol ethers) such as diethoxyethane and diethylene glycol dimethyl ether. The amount of the aprotic solvent A used with respect to the raw metal salt is preferably in a weight ratio range of 0.5 to 5. In addition, the raw metal salt may be isolated as a solid and dissolved in the aprotic solvent A, or the aprotic solvent A is added during hydrolysis and/or neutralization of perfluoro(2,4-dimethyl-2-fluoroformyl-1,3-dioxolane), and the solution is subjected to azeotropic distillation dehydration or the like to obtain a solution, which can be used.

The aprotic solvent B is the same as or different from the aprotic solvent A, and is a solvent having a boiling point equal to or higher than that of the aprotic solvent A. The target product perfluoro(2-methylene-4-methyl-1,3-dioxolane) can be obtained by a decarboxylation reaction of the raw metal salt. In order to cause a decarboxylation reaction of the raw metal salt at a sufficient reaction rate, it is desirable that the aprotic solvent B have a boiling point of equal to or higher than 130°C. In this regard, preferable examples of aprotic solvents B may include ether solvents (ethers) such as dibutyl ether, diphenyl ether, diethylene glycol dimethyl ether, triethylene glycol dimethyl ether, and tetraethylene glycol dimethyl ether, ester solvents (esters) such as ethyl octanoate, diethyl malonate, and diethyl succinate, ketoester solvents (ketoesters) such as ethyl acetoacetate, ketone solvents (ketones) such as cyclohexanone, amide solvents (amides) such as dimethylacetamide and N-methylpyrrolidone, nitrile solvents (nitriles) such as propiononitrile, aromatic hydrocarbon solvents (aromatic hydrocarbons) such as xylene and mesitylene, alkane solvents (alkanes) such as dodecane, decalin, and liquid paraffin, and mixed solvents of two or more thereof. Among these, in consideration of the yield of perfluoro(2-methylene-4-methyl-1,3-dioxolane), the aprotic solvent B preferably includes one or more solvents selected from the group consisting of alkane solvents and glycol diether solvents. The amount of the aprotic solvent B used with respect to the raw metal salt can be, for example, in a weight ratio range of 0.2 to 5.0.

Reaction distillation can be performed by continuously supplying a raw material solution containing a raw metal salt to a reaction distiller. The reaction distiller is not particularly limited as long as it is an instrument in which a raw material solution can be supplied, a product can be distilled (specifically, condensed by cooling) and as necessary, the pressure in the distiller can be reduced. Examples of such a reaction distiller include a three-neck round-bottom flask having a distiller (a cooler and a receiver) and a reaction vessel with a distillation instrument in a chemical plant.

Reaction distillation can be performed, for example, under atmospheric pressure or under a reduced pressure, and is preferably performed under a reduced pressure in a range of 1 kPa to 90 kPa. Preferably, under a reduced pressure, in the aprotic solvent B heated in the reaction container, the raw material solution can be continuously supplied. Here, the temperature of the aprotic solvent B in the reaction container is preferably in a temperature range of 100°C to 200°C. A rate of the raw material solution supplied into the reaction distiller is not particularly limited, and the rate at which the above temperature range can be maintained is preferable. Generally, in terms of weight/hour of the raw metal salt per 1 L of a reaction container capacity, the rate can be equal to or more than 1 g/hr and equal to or less than 100 g/hr and preferably, can be equal to or more than 10 g/hr and equal to or less than 60 g/hr. In addition, as necessary, after supply of the raw material solution is completed, under the same conditions, additional maintaining is performed for 0.5 hours to 4 hours, and distillation of the target product perfluoro(2-methylene-4-methyl-1,3-dioxolane) can be completed. The target product perfluoro(2-methylene-4-methyl-1,3-dioxolane) distillated by reaction distillation can be collected in a trap cooled to, for example, -20°C to -80°C. In addition, when depressurization is performed using a diaphragm pump, a pump outlet gas whose pressure has been increased to atmospheric pressure or higher is cooled to the boiling point of perfluoro(2-methylene-4-methyl-1,3-dioxolane) or lower, and thus the target product perfluoro(2-methylene-4-methyl-1,3-dioxolane) can also be collected.

According to the aspect B, as the target product perfluoro(2-methylene-4-methyl-1,3-dioxolane) distilled by reaction distillation, it is possible to obtain perfluoro(2-methylene-4-methyl-1,3-dioxolane) with a small amount of by-product 2-hydro-perfluoro(2,4-dimethyl-1,3-dioxolane) mixed in. Therefore, the product obtained by the above production method can be directly used as a synthetic raw material for poly[perfluoro(2-methylene-4-methyl-1,3-dioxolane)], or can be used as a synthetic raw material for poly[perfluoro(2-methylene-4-methyl-1,3-dioxolane)] after purification is performed by precision distillation as necessary.

### [Examples]

Hereinafter, the present invention will be described in detail with reference to Examples. However, the present invention is not limited to these Examples.

The following instrument was used for the analysis.
^{1 9} F-NMR: AVANCE II 400 (commercially available from BRUKER)

### <Reference Example 1>

### Preparation of perfluoro(2,4-dimethyl-2-fluoroformyl-1,3-dioxolane)

The following trifluoropyruvic acid fluoride dimers (2,222.4 g, a pure content of 1,980.3 g, 6.88 mol), cesium fluoride (315.6 g, 2.08 mol) and diethylene glycol dimethyl ether (1,338.8 g, 9.98 mol) were put into a 10 L SUS316 autoclave having a stirrer with a pressure resistance of 8 MPa and cooled on an ice bath at 0°C. Next, hexafluoropropylene oxide (2,284.4 g, 13.76 mol) was added thereto over 2 hours and then heated at 120°C, and the reaction was caused for 24 hours.

### Trifluoropyruvic acid fluoride dimer

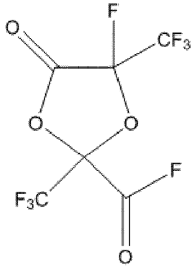

After the reaction was completed, cooling was performed to room temperature, liquid separation was performed, and crude perfluoro(2,4-dimethyl-2-fluoroformyl-1,3-dioxolane) was obtained (yellow liquid, 3,047.4 g). In quantification by ^{1 9} F-NMR using benzotrifluoride as an internal standard substance, 2,901.0 g (9.36 mol) of the following perfluoro(2,4-dimethyl-2-fluoroformyl-1,3-dioxolane) as a target product was produced (yield 68%/trifluoropyruvic acid fluoride dimer standard). The obtained target product was a mixture of two types of diastereomers at a ratio of 6/4 (molar ratio).

¹⁹F-NMR (neat, 376 MHz) (isomer 1) δ23.63, -77.76 (d, J=131.6 Hz), -80.13, -81.57, - 83.56 (d, J=135.4 Hz), -124.91. (isomer 2)δ23.16, -78.45 (d, J=131.6 Hz), -80.37, - 81.56, -84.05 (d, J=139.1 Hz), -123.72.

### Perfluoro(2,4-dimethyl-2-fluoroformyl-1,3-dioxolane

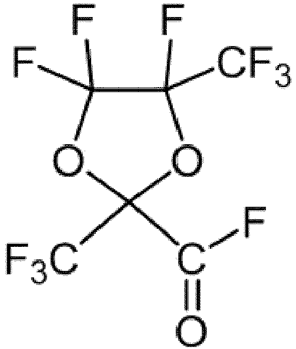

### <Reference Example 2>

### Preparation of perfluoro(2,4-dimethyl-1,3-dioxolane-2-yl)carboxylic acid potassium salt aqueous solution

Potassium carbonate (659.8 g, 4.77 mol) and water (1,400 mL) were put into a 10 L glass three-neck round-bottom flask having a stirrer and a dropping funnel and dissolved with stirring and then cooled on an ice bath at 0°C. Next, perfluoro(2,4-dimethyl-2-fluoroformyl-1,3-dioxolane) (1,510.2 g, pure content of 1,450.5 g, 4.68 mol) prepared in Reference Example 1 was added dropwise thereto over 2 hours while controlling heat generation in a temperature range of 0°C to 10°C, the mixture was then additionally stirred in the same temperature range for 1 hour and left for 1 hour, and a lower layer was then separated by a liquid separation operation, and 2,703 g of an aqueous solution containing the target product perfluoro(2,4-dimethyl-1,3-dioxolane-2-yl)carboxylic acid potassium salt was obtained (a pure content of 1,602.2 g, 4.63 mol, a yield of 99%).

### <Reference Example 3>

### Preparation of perfluoro(2,4-dimethyl-1,3-dioxolane-2-yl)carboxylic acid (solid)

The perfluoro(2,4-dimethyl-1,3-dioxolane-2-yl)carboxylic acid potassium salt aqueous solution (2,304 g, pure content of 1,365.7 g, 3.95 mol) obtained in Reference Example 2 was put into a 3 L flask, concentrated under a reduced pressure in an evaporator on a bath at 60°C at a pressure of 13 kPa, and then vacuum-dried at the same temperature for 5 hours, and 1,464.5 g of a white solid (a pure content of 1,357.8 g, 3.92 mol, a water content of 0.45%) was obtained.

¹⁹F -NMR (D₂O, 376 MHz) (isomer 1) δ-78.65 (dd, J=233.1 Hz, 11.3 Hz), -81.26 (d, J=11.3 Hz), -81.95, -84.44 (dd, J=135.4 Hz, 11.3 Hz), -124.42 (m). (isomer 2) δ-79.17 (dd, J=135.4 Hz, 7.5 Hz), -81.49 (d, J=15.0 Hz), -81.82, -84.57 (dd, J=142.9 Hz, 7.5 Hz), -124.78(m).

### <Reference Example 4>

### Preparation of hydrated perfluoro(2,4-dimethyl-1,3-dioxolane-2-yl)carboxylic acid sodium salt

Sodium carbonate (51.10 g, 0.482 mol) and water (150 mL) were put into a 1 L glass three-neck round-bottom flask having a stirrer and a dropping funnel, stirred and suspended, and then cooled on an ice bath at 0°C.

Next, 152.36 g of perfluoro(2,4-dimethyl-2-fluoroformyl-1,3-dioxolane) (a pure content of 145.00 g, 0.468 mol) prepared in Reference Example 1 was added dropwise thereto over 1 hour while controlling heat generation in a temperature range of 0°C to 10°C, the mixture was then additionally stirred in the same temperature range for 1 hour, and left for 1 hour, and a lower layer was then separated by a liquid separation operation, and 259.72 g of a target product perfluoro(2,4-dimethyl-1,3-dioxolane-2-yl)carboxylic acid sodium salt aqueous solution (a pure content of 146.74 g, 0.445 mol, a yield of 95%) was obtained.

### <Reference Example 5>

### Preparation of perfluoro(2,4-dimethyl-1,3-dioxolane-2-yl)carboxylic acid magnesium salt

Magnesium carbonate (40.64 g, 0.482 mol) and water (200 mL) were put into a 1 L glass three-neck round-bottom flask having a stirrer and a dropping funnel and made into a suspension with stirring, and then cooled on an ice bath at 0°C.

Next, perfluoro(2,4-dimethyl-2-fluoroformyl-1,3-dioxolane) (152.36 g, a pure content of 145.00 g, 0.468 mol) prepared in Reference Example 1 was added dropwise thereto over 1 hour while controlling heat generation in a temperature range of 0°C to 10°C, and the mixture was then additionally stirred at the same temperature for 1 hour and left for 1 hour, a lower layer was then separated by a liquid separation operation, and a target product hydrated bis[perfluoro(2,4-dimethyl-1,3-dioxolane-2-yl)carboxylic acid]magnesium salt aqueous solution 241.32 g (a pure content of 137.43 g, 0.215 mol, a yield of 92%) was obtained.

### [Examples and Comparative Examples of aspect A]

### <Example 1>

### Process (1): preparation of perfluoro(2,4-dimethyl-1,3-dioxolane-2-yl)carboxylic acid potassium salt aqueous solution

Potassium carbonate (65.65 g, 0.475 mol) and water (140 mL) were put into a 1 L glass three-neck round-bottom flask having a stirrer and a dropping funnel, and dissolved with stirring, and then cooled on an ice bath at 0°C. Next, perfluoro(2,4-dimethyl-2-fluoroformyl-1,3-dioxolane) (150.8 g, a pure content of 143.6 g, 0.463 mol) prepared in Reference Example 1 was added dropwise thereto over 1 hour while controlling heat generation in a temperature range of 0°C to 10°C, and the mixture was then additionally stirred in the same temperature range for 1 hour and left for 1 hour. A lower layer was separated by a liquid separation operation, and 268.3 g of a target product, which is perfluoro(2,4-dimethyl-1,3-dioxolane-2-yl)carboxylic acid potassium salt aqueous solution, was obtained. For the aqueous solution, quantitative analysis was performed by ¹⁹F-NMR using benzotrifluoride as an internal standard substance, and it was confirmed that 158.5 g (0.458 mol, a yield of 99%) of the perfluoro(2,4-dimethyl-1,3-dioxolane-2-yl)carboxylic acid potassium salt was contained and 11.96 g (0.206 mol) of potassium fluoride was contained.

### Process (2): preparation of perfluoro(2,4-dimethyl-1,3-dioxolane-2-yl)carboxylic acid potassium salt (solid)

The perfluoro(2,4-dimethyl-1,3-dioxolane-2-yl)carboxylic acid potassium salt aqueous solution (268.3 g, a pure content of 158.5 g, 0.458 mol) obtained in the process (1) was put into a 500 mL flask, concentrated under a reduced pressure in an evaporator on a bath at 60°C at a pressure of 13 kPa, and then vacuum-dried at the same temperature for 5 hours, and 170.2 g of a white solid (a pure content of 157.2 g, 30.454 mol, a water content of 0.43 weight%) was obtained.

¹⁹F-NMR (D₂ O, 376 MHz) (isomer 1) δ-78.65 (dd, J=233.1 Hz, 11.3 Hz), -81.26 (d, J=11.3 Hz), -81.95, -84.44 (dd, J=135.4 Hz, 11.3 Hz), -124.42 (m). (isomer 2)δ-79.17 (dd, J=135.4 Hz, 7.5 Hz), -81.49 (d, J=15.0 Hz), -81.82, -84.57 (dd, J=142.9 Hz, 7.5 Hz), -124.78(m).

### Process (3): synthesis of perfluoro(2-methylene-4-methyl-1,3-dioxolane)

The perfluoro(2,4-dimethyl-1,3-dioxolane-2-yl)carboxylic acid potassium salt (170.2 g, pure content 157.2 g, 0.454 mol) prepared in the process (2) and diethylene glycol dimethyl ether (255.3 g) were put into a 1 L glass three-neck round-bottom flask having a stirrer and a distillation device with a cold trap (two stages at -20°C and -78°C). Under a reduced pressure of 30 kPa, heating was gradually performed to 130°C with stirring, and the reaction was caused for 4 hours. After the reaction was completed, the product collected in the cold trap was analyzed by ¹⁹F-NMR using benzotrifluoride as an internal standard substance, and it was confirmed that 49.84 g (0.204 mol, a yield of 45%) of perfluoro(2-methylene-4-methyl-1,3-dioxolane) and 14.38 g (a yield of 12%) of 2-hydro-perfluoro(2,4-dimethyl-1,3-dioxolane) were produced.

### <Comparative Example 1>

The process (2) and the process (3) were performed in the same operation except that, in Example 1, the liquid separation operation was not performed in the process (1) and a total amount of the reaction solution was used.

That is, potassium carbonate (65.65 g, 0.475 mol) and water (140 mL) were put into a 1 L glass three-neck round-bottom flask having a stirrer and a dropping funnel, and dissolved with stirring, and then cooled on an ice bath at 0°C. Next, perfluoro(2,4-dimethyl-2-fluoroformyl-1,3-dioxolane) (150.8 g, a pure content of 143.6 g, 0.463 mol) prepared in Reference Example 1 was added dropwise thereto over 1 hour while controlling heat generation in a temperature range of 0°C to 10°C, the reaction solution was transferred to a 1 L flask, concentrated under a reduced pressure in an evaporator on a bath at 60°C at a pressure of 13 kPa, and then vacuum-dried in the same temperature range for 5 hours, and 187.5 g of a white solid (a pure content of 158.5 g, 0.458 mol, a water content of 1.36 weight%) was obtained. Next, perfluoro(2,4-dimethyl-1,3-dioxolane-2-yl)carboxylic acid potassium salt (187.5 g, a pure content of 158.5 g, 0.458 mol) prepared in the above (2) and diethylene glycol dimethyl ether (255.3 g) were put into a 1 L glass three-neck round-bottom flask having a stirrer and a distillation device with a cold trap (two stages at -20°C and 78°C). Under a reduced pressure of 30 kPa, heating was gradually performed to 130°C with stirring, and the reaction was caused for 4 hours.

After the reaction was completed, the product collected in the cold trap was analyzed by ¹⁹F-NMR using benzotrifluoride as an internal standard substance, and it was confirmed that 36.88 g (0.151 mol, a yield of 33%) of perfluoro(2-methylene-4-methyl-1,3-dioxolane) and 38.69 g (a yield of 32%) of 2-hydro-perfluoro(2,4-dimethyl-1,3-dioxolane) were produced.

### <Example 2>

### (Method in which an aprotic solvent C (diethylene glycol dimethyl ether) was added in the process (1))

### Process (1):

Potassium carbonate (65.64 g, 0.475 mol) and water (153.3 g) were put into a 1 L glass three-neck round-bottom flask having a stirrer and a dropping funnel, and dissolved with stirring, and diethylene glycol dimethyl ether (321.4 g) was then added, and cooled on an ice bath at 0°C. Next, perfluoro(2,4-dimethyl-2-fluoroformyl-1,3-dioxolane) (150.8 g, a pure content of 143.6 g, 0.463 mol) prepared in Reference Example 1 was added dropwise thereto over 1 hour while controlling heat generation in a temperature range of 0°C to 10°C, the mixture was then additionally stirred in the same temperature range for 1 hour and left for 1 hour. An upper layer was separated by a liquid separation operation, and 582.8 g of a solution containing a target product, which is perfluoro(2,4-dimethyl-1,3-dioxolane-2-yl)carboxylic acid potassium salt, was obtained. For the solution, quantitative analysis was performed by ^{1 9} F-NMR using benzotrifluoride as an internal standard substance, and it was confirmed that 159.4 g (0.461 mol, a yield of 100%) of perfluoro(2,4-dimethyl-1,3-dioxolane-2-yl)carboxylic acid potassium salt was contained and 1.72 g (0.030 mol) of potassium fluoride was contained.

### Process (2):

The perfluoro(2,4-dimethyl-1,3-dioxolane-2-yl)carboxylic acid potassium salt solution (582.8 g, a pure content of 159.4 g, 0.461 mol) obtained in the process (1) was put into a 1 L flask having a stirrer and a distillation device, and while gradually reducing the pressure from 13 kPa to 1 kPa on an oil bath at 60°C, water and diethylene glycol dimethyl ether were distilled off, and a perfluoro(2,4-dimethyl-1,3-dioxolane-2-yl)carboxylic acid potassium salt solution (386.9 g, a pure content of 154.7 g, 0.447 mol, a water content of 0.058 weight%) was obtained.

### Process (3):

The perfluoro(2,4-dimethyl-1,3-dioxolane-2-yl)carboxylic acid potassium salt solution (386.9 g, a pure content of 154.7 g, 0.447 mol) prepared in the process (2) was put into a 1 L glass three-neck round-bottom flask having a stirrer and a distillation device with a cold trap (two stages at -20°C and -78°C). Under a reduced pressure of 30 kPa, heating was gradually performed to 130°C with stirring, and the reaction was caused for 4 hours. After the reaction was completed, the product collected in the cold trap was analyzed by ¹⁹F-NMR using benzotrifluoride as an internal standard substance, and it was confirmed that 57.81 g (0.237 mol, a yield of 53%) of perfluoro(2-methylene-4-methyl-1,3-dioxolane) and 9.44 g (a yield of 8%) of 2-hydro-perfluoro(2,4-dimethyl-1,3-dioxolane) were produced.

### <Example 3>

### (Method in which, in the process (1), an organic layer was brought into contact with an inorganic salt aqueous solution (30 weight% potassium carbonate aqueous solution) and liquid separation was then performed again)

### Process (1):

Potassium carbonate (65.66 g, 0.475 mol) and water (153.1 g) were put into a 1 L glass three-neck round-bottom flask having a stirrer and a dropping funnel and dissolved with stirring, and then cooled on an ice bath at 0°C. Next, perfluoro(2,4-dimethyl-2-fluoroformyl-1,3-dioxolane) (150.8 g, a pure content of 143.6 g, 0.463 mol) prepared in Reference Example 1 was added dropwise thereto over 1 hour while controlling heat generation in a temperature range of 0°C to 10°C, and the mixture was then additionally stirred in the same temperature range for 1 hour and left for 1 hour, and a lower layer was then separated by a liquid separation operation.

### Process (4): re-separating process

The separated lower layer was put into a 1 L flask again, 150.4 g of diethylene glycol dimethyl ether and a 30 weight% potassium carbonate aqueous solution (145.9 g) were added, and the mixture was stirred for 1 hour and left for 1 hour. An upper layer was separated off by a liquid separation operation, and 422.1 g of a perfluoro(2,4-dimethyl-1,3-dioxolane-2-yl)carboxylic acid potassium salt solution was obtained. For the solution, quantitative analysis was performed by ¹⁹F-NMR using benzotrifluoride as an internal standard substance, and it was confirmed that 158.5 (0.458 mol, a yield of 99%) of the perfluoro(2,4-dimethyl-1,3-dioxolane-2-yl)carboxylic acid potassium salt was contained and 0.95 g (0.016 mol) of potassium fluoride was contained.

### Process (2):

The perfluoro(2,4-dimethyl-1,3-dioxolane-2-yl)carboxylic acid potassium salt solution (422.1 g, a pure content of 158.5 g, 0.458 mol) obtained in the process (1) was put into a 1 L flask having a stirrer and a distillation device, and while gradually reducing the pressure from 13 kPa to 1 kPa on an oil bath at 60°C, water and diethylene glycol dimethyl ether were distilled off, and 150.3 g of diethylene glycol was then added again, and evaporated under a reduced pressure of 1 kPa, and a perfluoro(2,4-dimethyl-1,3-dioxolane-2-yl)carboxylic acid potassium salt solution (390.4 g, a pure content of 152.3 g, 0.440 mol, a water content of 0.046 weight%) was obtained.

### Process (3):

The perfluoro(2,4-dimethyl-1,3-dioxolane-2-yl)carboxylic acid potassium salt solution (390.4 g, a pure content of 152.3 g, 0.440 mol) prepared in the process (2) was put into a 1 L glass three-neck round-bottom flask having a stirrer and a distillation device with a cold trap (two stages at -20°C and -78°C). Under a reduced pressure of 30 kPa, heating was gradually performed to 130°C with stirring, and the reaction was caused for 4 hours. After the reaction was completed, the product collected in the cold trap was analyzed by ¹⁹F-NMR using benzotrifluoride as an internal standard substance, and it was confirmed that 59.05 g (0.242 mol, a yield of 55%) of perfluoro(2-methylene-4-methyl-1,3-dioxolane) and 8.13 g (a yield of 7%) of 2-hydro-perfluoro(2,4-dimethyl-1,3-dioxolane) were produced.

### <Example 4>

### (Method in which an aprotic solvent C (diethylene glycol dimethyl ether) was added in the process (1) and toluene was used as an azeotropic solvent in the process (2)) Process (1):

Potassium carbonate (65.64 g, 0.475 mol) and water (153.3 g) were put into a 1 L glass three-neck round-bottom flask having a stirrer and a dropping funnel and dissolved with stirring, and diethylene glycol dimethyl ether (150.1 g) was then added and cooled on an ice bath at 0°C. Next, perfluoro(2,4-dimethyl-2-fluoroformyl-1,3-dioxolane) (150.8 g, a pure content of 143.6 g, 0.463 mol) prepared in Reference Example 1 was added dropwise thereto over 1 hour while controlling heat generation in a temperature range of 0°C to 10°C, and the mixture was then additionally stirred in the same temperature range for 1 hour and left for 1 hour. An upper layer was separated by a liquid separation operation, and 418.4 g of a solution containing a target product, which is perfluoro(2,4-dimethyl-1,3-dioxolane-2-yl)carboxylic acid potassium salt, was obtained. For the solution, quantitative analysis was performed by ¹⁹F-NMR using benzotrifluoride as an internal standard substance, and it was confirmed that 159.9 g (0.462 mol, a yield of 100%) of perfluoro(2,4-dimethyl-1,3-dioxolane-2-yl)carboxylic acid potassium salt was contained and 1.87 g (0.032 mol) of potassium fluoride was contained.

### Process (2):

The perfluoro(2,4-dimethyl-1,3-dioxolane-2-yl)carboxylic acid potassium salt solution (418.4 g, a pure content of 159.9 g, 0.462 mol) obtained in the process (1) was put into a 1 L flask having a stirrer and a distillation device, the pressure was gradually reduced to 6 kPa on an oil bath at 50°C, and water was distilled off. Then, toluene (150.2 g) was added, and again while reducing the pressure to 6 kPa on a bath at 50°C, an azeotropic mixture of water and toluene was distilled off, and a perfluoro(2,4-dimethyl-1,3-dioxolane-2-yl)carboxylic acid potassium salt solution (351.8 g, a pure content of 158.3 g, 0.457 mol, a water content of 0.058 weight%) was obtained.

### Process (3):

The perfluoro(2,4-dimethyl-1,3-dioxolane-2-yl)carboxylic acid potassium salt solution (351.8 g, a pure content of 158.3 g, 0.457 mol) prepared in the process (2) was put into a 1 L glass three-neck round-bottom flask having a stirrer and a distillation device with a cold trap (two stages at -20°C and -78°C). Under a reduced pressure of 30 kPa, heating was gradually performed to 130°C with stirring, and the reaction was caused for 4 hours. After the reaction was completed, the product collected in the cold trap was analyzed by ¹⁹F-NMR using benzotrifluoride as an internal standard substance, and it was confirmed that 57.98 g (0.238 mol, a yield of 52%) of perfluoro(2-methylene-4-methyl-1,3-dioxolane) and 10.86 g (a yield of 9%) of 2-hydro-perfluoro(2,4-dimethyl-1,3-dioxolane) were produced.

### <Example 5>

### (Method in which a perfluoro(2,4-dimethyl-1,3-dioxolane-2-yl)carboxylic acid potassium salt solution was continuously added dropwise in the process (3)) Process (1):

Potassium carbonate (65.64 g, 0.475 mol) and water (153.3 g) were put into a 1 L glass three-neck round-bottom flask having a stirrer and a dropping funnel, and dissolved with stirring, and diethylene glycol dimethyl ether (320.5 g) was then added, and cooled on an ice bath at 0°C. Next, perfluoro(2,4-dimethyl-2-fluoroformyl-1,3-dioxolane) (150.8 g, a pure content of 143.6 g, 0.463 mol) prepared in Reference Example 1 was added dropwise thereto over 1 hour while controlling heat generation in a temperature range of 0°C to 10°C, and the mixture was then additionally stirred in the same temperature range for 1 hour and left for 1 hour, an upper layer was then separated by a liquid separation operation, and 580.5 g of solution containing a target product, which is perfluoro(2,4-dimethyl-1,3-dioxolane-2-yl)carboxylic acid potassium salt, was obtained. For the solution, quantitative analysis was performed by ¹⁹F-NMR using benzotrifluoride as an internal standard substance, and it was confirmed that 159.2 (0.460 mol, a yield of 100%) of a perfluoro(2,4-dimethyl-1,3-dioxolane-2-yl)carboxylic acid potassium salt was contained and 1.68 g (0.029 mol) of potassium fluoride was contained.

### Process (2):

The perfluoro(2,4-dimethyl-1,3-dioxolane-2-yl)carboxylic acid potassium salt solution (580.5 g, a pure content of 159.2 g, 0.460 mol) obtained in the process (1) was put into a 1 L flask having a stirrer and a distillation device and while gradually reducing the pressure in a range of 13 kPa to 1 kPa on an oil bath at 60°C, water and diethylene glycol dimethyl ether were distilled off, and a perfluoro(2,4-dimethyl-1,3-dioxolane-2-yl)carboxylic acid potassium salt solution (387.6 g, a pure content of 154.7 g, 0.447 mol, a water content of 0.052 weight%) was obtained.

### Process (3):

Liquid paraffin (commercially available from FUJIFILM Wako Pure Chemical Corporation, an initial boiling point of 300°C, 158 g) was put into a 1 L glass three-neck round-bottom flask having a stirrer and a distillation device with a cold trap (two stages at -20°C and -78°C), and under a reduced pressure of 30 kPa, heating was performed to 130°C with stirring, and the perfluoro(2,4-dimethyl-1,3-dioxolane-2-yl)carboxylic acid potassium salt solution (386.9 g, a pure content of 154.7 g, 0.447 mol) prepared in the process (2) was then added dropwise and reacted for 3 hours, and the mixture was additionally left under the same reduced pressure and at the same temperature for 1 hour. After the reaction was completed, the product collected in the cold trap was analyzed by ¹⁹F-NMR using benzotrifluoride as an internal standard substance, and it was confirmed that 85.07 g (0.349 mol, a yield of 78%) of perfluoro(2-methylene-4-methyl-1,3-dioxolane) and 4.72 g (a yield of 4%) of 2-hydro-perfluoro(2,4-dimethyl-1,3-dioxolane) were produced.

### <Example 6>

### (Method in which a sodium salt was used as a perfluoro(2,4-dimethyl-1,3-dioxolane-2-yl)carboxylic acid alkali metal salt)

### Process (1):

Sodium carbonate (51.10 g, 0.482 mol) and water (150 mL) were put into a 1 L glass three-neck round-bottom flask having a stirrer and a dropping funnel, stirred and suspended, and then cooled on an ice bath at 0°C.

Next, 152.36 g (a pure content of 145.00 g, 0.468 mol) of perfluoro(2,4-dimethyl-2-fluoroformyl-1,3-dioxolane) prepared in Reference Example 1 was added dropwise thereto over 1 hour while controlling heat generation in a temperature range of 0°C to 10°C, the mixture was then additionally stirred in the same temperature range for 1 hour and left for 1 hour, a lower layer was then separated by a liquid separation operation, and 259.72 g (a pure content of 146.74 g, 0.445 mol, a yield of 95%) of a target product, which is perfluoro(2,4-dimethyl-1,3-dioxolane-2-yl)carboxylic acid sodium salt solution, was obtained. The content of sodium fluoride was 4.56 g (0.109 mol).

### Process (2):

259.72 g (146.74 g, 0.445 mol) of the perfluoro(2,4-dimethyl-1,3-dioxolane-2-yl)carboxylic acid sodium salt aqueous solution obtained in the process (1) and diethylene glycol dimethyl ether (300 g) were put into a 1 L glass three-neck round-bottom flask having a stirrer and a distillation device, heated on an oil bath at 60°C, and the contained water was azeotropically evaporated with diethylene glycol dimethyl ether at a pressure of 13 kPa to 1 kPa while gradually increasing the degree of decompression, and 320.60 g of a diethylene glycol dimethyl ether solution containing the perfluoro(2,4-dimethyl-1,3-dioxolane-2-yl)carboxylic acid sodium salt was obtained (a target product content of 145.88 g, 0.442 mol, a water content of 0.09 weight%).

### Process (3):

Liquid paraffin (commercially available from FUJIFILM Wako Pure Chemical Corporation, an initial boiling point of 300°C, 146 g) was put into a 1 L glass three-neck round-bottom flask having a stirrer, a dropping funnel and a distillation device with a cold trap (two stages at -20°C and -78°C), and under a reduced pressure of 30 kPa, heating was performed to 135°C with stirring, and the diethylene glycol dimethyl ether solution (320.6 g) containing the perfluoro(2,4-dimethyl-1,3-dioxolane-2-yl)carboxylic acid sodium salt prepared in the process (2) was then added dropwise thereto over 3 hours, and the mixture was additionally left under the same reduced pressure and at the same temperature for 1 hour. After the reaction was completed, the product collected in the cold trap (78.02 g) was analyzed by ¹⁹F-NMR using benzotrifluoride as an internal standard substance, and it was confirmed that 73.34 g (0.301 mol, a yield of 68%) of perfluoro(2-methylene-4-methyl-1,3-dioxolane) was produced. The amount of 2-hydro-perfluoro(2,4-dimethyl-1,3-dioxolane) produced was 4.55 g (a yield of 3.9%).

### <Example 7>

### (Method in which a magnesium salt was used as a perfluoro(2,4-dimethyl-1,3-dioxolane-2-yl)carboxylic acid alkaline earth metal salt)

### Process (1):

Magnesium carbonate (40.64 g, 0.482 mol) and water (200 mL) were put into a 1 L glass three-neck round-bottom flask having a stirrer and a dropping funnel, and made into a suspension with stirring and then cooled on an ice bath at 0°C.

Next, perfluoro(2,4-dimethyl-2-fluoroformyl-1,3-dioxolane) (152.36 g, a pure content of 145.00 g, 0.468 mol) prepared in Reference Example 1 was added dropwise thereto over 1 hour while controlling heat generation in a temperature range of 0°C to 10°C, and the mixture was then additionally stirred in the same temperature range for 1 hour and left for 1 hour, a lower layer was then separated by a liquid separation operation, and 241.32 g of a target product, which is hydrated bis[perfluoro(2,4-dimethyl-1,3-dioxolane-2-yl)carboxylic acid]magnesium salt solution, was obtained (a pure content of 137.43 g, 0.215 mol, a yield of 92%). The content of magnesium fluoride was 1.32 g (0.021 mol).

### Process (2):

241.32 g of the bis[perfluoro(2,4-dimethyl-1,3-dioxolane-2-yl)carboxylic acid]magnesium salt solution (137.43 g, 0.215 mol, a yield of 92%) obtained in the process (1) and diethylene glycol dimethyl ether (300 g) were put into a 1 L glass three-neck round-bottom flask having a stirrer and a distillation device, heated on an oil bath at 60°C, and the contained waterwas azeotropically evaporated with diethylene glycol dimethyl ether at a pressure of 13 kPa to 1 kPa while gradually increasing the degree of decompression, and 382.41 g of the diethylene glycol dimethyl ether solution (a target product content of 136.20 g, 0.213 mol, a water content of 0.07 weight%) containing a bis[perfluoro(2,4-dimethyl-1,3-dioxolane-2-yl)carboxylic acid]magnesium salt was obtained.

### Process (3):

Liquid paraffin (commercially available from FUJIFILM Wako Pure Chemical Corporation, an initial boiling point of 300°C, 136 g) was put into a 1 L glass three-neck round-bottom flask having a stirrer, a dropping funnel and a distillation device with a cold trap (two stages at -20°C and -78°C), and under a reduced pressure of 30 kPa, heating was performed to 140°C with stirring, a diethylene glycol dimethyl ether solution (382.4 g) containing the bis[perfluoro(2,4-dimethyl-1,3-dioxolane-2-yl)carboxylic acid]magnesium salt was then added dropwise thereto over 3 hours, and the mixture was additionally left under the same reduced pressure and at the same temperature for 1 hour. After the reaction was completed, the product collected in the cold trap (74.24 g) was analyzed by ¹⁹F-NMR using benzotrifluoride as an internal standard substance, and it was confirmed that 67.56 g (0.277 mol, a yield of 65%) of perfluoro(2-methylene-4-methyl-1,3-dioxolane) was produced. The amount of 2-hydro-perfluoro(2,4-dimethyl-1,3-dioxolane) produced was 4.50 g (a yield of 4.0%).

In Examples 1 to 7, perfluoro(2-methylene-4-methyl-1,3-dioxolane) was obtained according to the decarboxylation reaction in the liquid phase system, which is easy to perform on an industrial scale. Comparing Examples 1 to 7 with Comparative Example 1, compared to Comparative Example 1, in Examples 1 to 7, it was confirmed that perfluoro(2-methylene-4-methyl-1,3-dioxolane) was obtained with a higher yield and production of 2-hydro-perfluoro(2,4-dimethyl-1,3-dioxolane) as a by-product was suppressed.

### [Examples and Comparative Examples of aspect B]

### <Example 8>

### Synthesis of perfluoro(2-methylene-4-methyl-1,3-dioxolane)

The perfluoro(2,4-dimethyl-1,3-dioxolane-2-yl)carboxylic acid potassium salt aqueous solution (270 g, a pure content of 160.2 g, 0.463 mol) obtained in Reference Example 2, diethylene glycol dimethyl ether (320 g) and a 30 weight% potassium carbonate aqueous solution (120 g) were put into a 1 L three-neck round-bottom flask, and the mixture was stirred for 0.5 hours and left for 0.5 hours, and an upper layer was then separated from the reaction product separated into two layers by a liquid separation operation.

Next, the separated upper layer was put into a 1 L glass three-neck round-bottom flask having a stirrer and a distillation device and heated on an oil bath at 60°C, and the contained water was azeotropically evaporated with diethylene glycol dimethyl ether at a pressure of 13 kPa to 1 kPa while gradually increasing the degree of decompression, and 411.5 g of a diethylene glycol dimethyl ether solution containing a perfluoro(2,4-dimethyl-1,3-dioxolane-2-yl)carboxylic acid potassium salt was obtained (a target product content of 158.8 g, 0.459 mol, a water content of 0.06 weight%).

Next, liquid paraffin (commercially available from FUJIFILM Wako Pure Chemical Corporation, an initial boiling point of 300°C, 159 g) was put into a 1 L glass three-neck round-bottom flask having a stirrer, a dropping funnel and a distillation device with a cold trap (two stages at -20°C and -78°C), and under a reduced pressure of 30 kPa, heating was performed to 130°C with stirring, a diethylene glycol dimethyl ether solution (411.5 g) containing the perfluoro(2,4-dimethyl-1,3-dioxolane-2-yl)carboxylic acid potassium salt was then added dropwise thereto over 3 hours, and the mixture was additionally left under the same reduced pressure and at the same temperature for 1 hour. After the reaction was completed, the product collected in the cold trap (99.66 g) was analyzed by ¹⁹F-NMR using benzotrifluoride as an internal standard substance, and thus it was confirmed that 92.96 g (0.381 mol, a yield of 83%) of perfluoro(2-methylene-4-methyl-1,3-dioxolane) was produced. The amount of 2-hydro-perfluoro(2,4-dimethyl-1,3-dioxolane) produced was 3.64 g (a yield of 3.0%).

The product collected in the cold trap was put into a 200 mL round-bottom flask having a distillation device composed of rectifying columns of 10 steps of Kiriyama Pac and a stirring bar, and precision distillation was performed under atmospheric pressure at a distribution ratio of 10/1 to obtain purified perfluoro(2-methylene-4-methyl-1,3-dioxolane) (a boiling point of 43°C to 44°C, 76.2 g, a purity of 98.2%). ¹⁹F-NMR (CDCl₃, 376 MHz) δ-82.08(m), -83.90 (dq, J=136.3 Hz, 7.5 Hz), -90.30 (dq, J=136.3 Hz, 5.0 Hz), -126.78 (d, J=124.1 Hz), -128.13 (dquin, J=124.1 Hz, 1.0 Hz), - 130.48(m).

### <Example 9>

### Synthesis of perfluoro(2-methylene-4-methyl-1,3-dioxolane)

Liquid paraffin(commercially available from FUJIFILM Wako Pure Chemical Corporation, an initial boiling point of 300°C, 159 g) was put into a 1 L glass three-neck round-bottom flask having a stirrer, a dropping funnel and a distillation device with a cold trap (two stages at -20°C and -78°C), and under a reduced pressure of 30 kPa, heating was performed to 130°C with stirring. The perfluoro(2,4-dimethyl-1,3-dioxolane-2-yl)carboxylic acid potassium salt (171.3 g, a pure content of 158.8 g, 0.459 mol) obtained in Reference Example 3 was dissolved in diethylene glycol dimethyl ether (238.2 g) and this was added dropwise from the dropping funnel over 3 hours, and the mixture was additionally left under the same reduced pressure and at the same temperature for 1 hour. After the reaction was completed, the product collected in the cold trap was analyzed by ¹⁹F-NMR using benzotrifluoride as an internal standard substance, and thus it was confirmed that 87.35 g (0.358 mol, a yield of 78%) of perfluoro(2-methylene-4-methyl-1,3-dioxolane) was produced. The amount of 2-hydro-perfluoro(2,4-dimethyl-1,3-dioxolane) produced was 4.12 g (a yield of 3.4%).

### <Comparative Example 2>

### Synthesis of perfluoro(2-methylene-4-methyl-1,3-dioxolane)

The perfluoro(2,4-dimethyl-1,3-dioxolane-2-yl)carboxylic acid potassium salt (171.3 g, a pure content of 158.8 g, 0.459 mol) prepared in Reference Example 3 and diethylene glycol dimethyl ether (238.2 g) were put into a 1 L glass three-neck round-bottom flask having a stirrer and a distillation device with a cold trap (two stages at - 20°C and -78°C). Under a reduced pressure of 30 kPa, heating was gradually performed to 130°C with stirring, and the reaction was caused for 4 hours. After the reaction was completed, the product collected in the cold trap was analyzed by ¹⁹F-NMR using benzotrifluoride as an internal standard substance, and thus it was confirmed that 49.28 g (0.202 mol, a yield of 44%) of perfluoro(2-methylene-4-methyl-1,3-dioxolane) and 15.75 g (a yield of 13%) of 2-hydro-perfluoro(2,4-dimethyl-1,3-dioxolane) were produced.

### <Examples 10 to 15>

### Synthesis of perfluoro(2-methylene-4-methyl-1,3-dioxolane)

The reaction was caused in the same method as in Example 9 except that the aprotic solvent A, the aprotic solvent B, and the reaction conditions were changed to solvents and conditions shown in Table 1. The product collected in the cold trap was analyzed by ^{1 9} F-NMR analysis using benzotrifluoride as an internal standard substance, and the yields of perfluoro(2-methylene-4-methyl-1,3-dioxolane) and 2-hydro-perfluoro(2,4-dimethyl-1,3-dioxolane) were calculated.

**[Table 1]**

| | Aprotic solvent A boiling point /°C | Aprotic solvent A /PPDD (weight ratio) | Aprotic solvent B (boiling point /°C) | Aprotic solvent BI PPDD (weight ratio) | Reaction pressure (kPa) | Reaction temperature (°C) | PMMD yield (%) | PDHD yield (%) |
|---|---|---|---|---|---|---|---|---|
| Example 9 | Diethylene glycol dimethyl ether 162°C | 1.5 | Liquid paraffin 300°C (initial boiling point) | 1.0 | 30 | 130 | 78 | 3.4 |
| Example 10 | Diethylene glycol dimethyl ether 162°C | 1.5 | Diethylene glycol dimethyl ether 162°C | 0.5 | 30 | 130 | 71 | 1.7 |
| Example 11 | Diethylene glycol dimethyl ether 162°C | 1.5 | Tetraethylene glycol dimethyl ether 276°C | 1.0 | 20 | 125 | 79 | 2.8 |
| Example 12 | Diethylene glycol dimethyl ether 162°C | 1.5 | n-dodecane 215°C | 1.0 | 30 | 130 | 67 | 3.1 |
| Example 13 | Di-n-butyl ether 142°C | 4.0 | Liquid paraffin 300°C(initial boiling point) | 1.0 | 50 | 130 | 65 | 2.9 |
| Example 14 | Dimethylacetamide 165°C | 1.5 | Liquid paraffin 300°C(initial boiling point) | 1.0 | 30 | 130 | 56 | 7.6 |
| Example 15 | Diethylene glycol dimethyl ether 162°C | 1.5 | Liquid paraffin 300°C(initial boiling point) | 1.0 | 40 | 140 | 75 | 3.3 |

| | | | | | | | | |
|---|---|---|---|---|---|---|---|---|
| Potassium perfluoro (2,4-dimethyl-1,3-dioxolane-2-yl) carboxylic acid: PPDD Perfluoro (2-methylene-4-methyl-1,3-dioxolane): PMMD 2-hydro-perfluoro(2,4-dimethyl-1,3-dioxolane): PDHD | | | | | | | | |

### <Example 16>

### Synthesis of perfluoro(2-methylene-4-methyl-1,3-dioxolane)

259.72 g (146.74 g, 0.445 mol) of the perfluoro(2,4-dimethyl-1,3-dioxolane-2-yl)carboxylic acid sodium salt aqueous solution obtained in Reference Example 4 and diethylene glycol dimethyl ether (300 g) were put into a 1 L glass three-neck round-bottom flask having a stirrer and a distillation device and heated on an oil bath at 60°C, and the contained water was azeotropically evaporated with diethylene glycol dimethyl ether at a pressure of 13 kPa to 1 kPa while gradually increasing the degree of decompression, and 320.60 g of a diethylene glycol dimethyl ether solution containing a perfluoro(2,4-dimethyl-1,3-dioxolane-2-yl)carboxylic acid sodium salt was obtained (a target product content of 145.88 g, 0.442 mol). Next, liquid paraffin (commercially available from FUJIFILM Wako Pure Chemical Corporation, an initial boiling point of 300°C, 146 g) was put into a 1 L glass three-neck round-bottom flask having a stirrer, a dropping funnel and a distillation device with a cold trap (two stages at -20°C and -78°C), and under a reduced pressure of 30 kPa, heating was performed to 135°C with stirring, and a diethylene glycol dimethyl ether solution (320.6 g) containing the perfluoro(2,4-dimethyl-1,3-dioxolane-2-yl)carboxylic acid sodium salt was then added dropwise thereto over 3 hours, and the mixture was additionally left under the same reduced pressure and at the same temperature for 1 hour. After the reaction was completed, the product collected in the cold trap (78.02 g) was analyzed by ¹⁹F-NMR using benzotrifluoride as an internal standard substance, and thus it was confirmed that 73.34 g (0.301 mol, a yield of 68%) of perfluoro(2-methylene-4-methyl-1,3-dioxolane) was produced. The amount of 2-hydro-perfluoro(2,4-dimethyl-1,3-dioxolane) produced was 4.55 g (a yield of 3.9%).

### <Example 17>

### Synthesis of perfluoro(2-methylene-4-methyl-1,3-dioxolane)

241.32 g (137.43 g, 0.215 mol) of the bis[perfluoro(2,4-dimethyl-1,3-dioxolane-2-yl)carboxylic acid]magnesium salt aqueous solution obtained in Reference Example 5 and diethylene glycol dimethyl ether (300 g) were put into a 1 L glass three-neck round-bottom flask having a stirrer and a distillation device and heated on an oil bath at 60°C, and the contained water was azeotropically evaporated with diethylene glycol dimethyl ether at a pressure of 13 kPa to 1 kPa while gradually increasing the degree of decompression, and 382.41 g (a target product content of 136.20 g, 0.213 mol) of a diethylene glycol dimethyl ether solution containing a bis[perfluoro(2,4-dimethyl-1,3-dioxolane-2-yl)carboxylic acid]magnesium salt was obtained.

Next, liquid paraffin (commercially available from FUJIFILM Wako Pure Chemical Corporation, an initial boiling point of 300°C, 136 g) was put into a 1 L glass three-neck round-bottom flask having a stirrer, a dropping funnel and a distillation device with a cold trap (two stages at -20°C and -78°C), and under a reduced pressure of 30 kPa, heating was performed to 140°C with stirring, and a diethylene glycol dimethyl ether solution (382.4 g) containing the bis[perfluoro(2,4-dimethyl-1,3-dioxolane-2-yl)carboxylic acid]magnesium salt was then added dropwise thereto over 3 hours, and the mixture was additionally left under the same reduced pressure and at the same temperature for 1 hour. After the reaction was completed, the product collected in the cold trap (74.24 g) was analyzed by ¹⁹F-NMR using benzotrifluoride as an internal standard substance, and thus it was confirmed that 67.56 g (0.277 mol, a yield of 65%) of perfluoro(2-methylene-4-methyl-1,3-dioxolane) was produced. The amount of 2-hydro-perfluoro(2,4-dimethyl-1,3-dioxolane) produced was 4.50 g (a yield of 4.0%).

In Examples 8 to 17, perfluoro(2-methylene-4-methyl-1,3-dioxolane) was obtained according to the decarboxylation reaction in the liquid phase system, which is easy to perform on an industrial scale. Comparing Examples 8 to 17 with Comparative Example 2, it was confirmed that, in Examples 8 to 17, perfluoro(2-methylene-4-methyl-1,3-dioxolane) was obtained with a yield equal to or higher than that of Comparative Example 2 in which the decarboxylation reaction was caused in a solid phase system, and production of 2-hydro-perfluoro(2,4-dimethyl-1,3-dioxolane) as a by-product was suppressed to the same extent or less than that of Comparative Example 2.

According to the various aspects described above, it is possible to produce perfluoro(2-methylene-4-methyl-1,3-dioxolane) on an industrial scale. The perfluoro(2-methylene-4-methyl-1,3-dioxolane) obtained in this manner can be used as a synthetic raw material for poly[(perfluoro(2-methylene-4-methyl-1,3-dioxolane)] being prospective as a resin for a gas separation membrane and a transparent resin for optical fibers.

This application claims the benefit of priority to Japanese Patent Application No. 2018-208937 filed on November 6, 2018 and Japanese Patent Application No. 2018-208938 filed on November 6, 2018, which are expressly incorporated herein by reference in their entirety.

## Claims

1. A method of producing perfluoro(2-methylene-4-methyl-1,3-dioxolane), the method comprising at least following processes (1) to (3):
(1) reacting at least one of perfluoro(2,4-dimethyl-2-fluoroformyl-1,3-dioxolane) and a hydrolysis product thereof with a basic aqueous solution containing one or more cations selected from the group consisting of alkali metal ions and alkaline earth metal ions and then separating a liquid containing produced perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts by a liquid separation operation;
(2) performing one or more water content reduction treatments selected from the group consisting of water evaporation and water adsorption on the liquid containing the obtained perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts to obtain perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts in a solution state or a solid state; and
(3) causing a decarboxylation reaction in a liquid phase system with the obtained perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts in a solution state or a solid state to produce perfluoro(2-methylene-4-methyl-1,3-dioxolane).

2. The method of producing perfluoro(2-methylene-4-methyl-1,3-dioxolane) according to claim 1, further comprising a following process (4) after the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts is separated by the liquid separation operation in the process (1):
(4) performing an operation at least once in which the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts obtained in the process (1) is additionally brought into contact with water or water in which an inorganic salt is dissolved, a liquid separation operation is then performed such that the liquid containing the perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts is again separated.

3. The method of producing perfluoro(2-methylene-4-methyl-1,3-dioxolane) according to claim 1 or 2, further comprising adding one or more aprotic solvents C at least any time from among: before the basic aqueous solution containing one or more cations selected from the group consisting of alkali metal ions and alkaline earth metal ions is reacted; after the reaction; and after the liquid separation operation in the process (1).

4. The method of producing perfluoro(2-methylene-4-methyl-1,3-dioxolane) according to claim 3,
wherein the aprotic solvent C is an ether solvent.

5. The method of producing perfluoro(2-methylene-4-methyl-1,3-dioxolane) according to any one of claims 1 to 4,
wherein the water content reduction treatment in the process (2) comprises water evaporation in the presence of one or more azeotropic solvents that form an azeotropic composition with water.

6. The method of producing perfluoro(2-methylene-4-methyl-1,3-dioxolane) according to any one of claims 3 to 4,
wherein the water content reduction treatment in the process (2) comprises water evaporation in the presence of one or more azeotropic solvents which form an azeotropic composition with water and have a boiling point lower than that of the aprotic solvent C which is added in the process (1).

7. The method of producing perfluoro(2-methylene-4-methyl-1,3-dioxolane) according to claim 5 or 6,
wherein the azeotropic solvent is one or more solvents selected from the group consisting of isopropyl alcohol and toluene.

8. The method of producing perfluoro(2-methylene-4-methyl-1,3-dioxolane) according to any one of claims 1 to 7,
wherein the solvent contained in the liquid phase system when the decarboxylation reaction is caused in the liquid phase system in the process (3) comprises a glycol diether solvent.

9. A method of producing perfluoro(2-methylene-4-methyl-1,3-dioxolane), the method comprising a reaction distillation process in which reaction distillation is performed by continuously supplying, to a reaction distiller, a raw material solution containing perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts to produce perfluoro(2-methylene-4-methyl-1,3-dioxolane).

10. The method of producing perfluoro(2-methylene-4-methyl-1,3-dioxolane) according to claim 9,
wherein the reaction distillation process comprises continuous supply of a raw material solution of perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts dissolved in an aprotic solvent A to an aprotic solvent B heated in the reaction distiller under a reduced pressure and performing reaction while distilling off the produced perfluoro(2-methylene-4-methyl-1,3-dioxolane) to the outside of the system, and
the aprotic solvent B is a solvent which is the same as or different from the aprotic solvent A and has a boiling point equal to or higher than that of the aprotic solvent A.

11. The method of producing perfluoro(2-methylene-4-methyl-1,3-dioxolane) according to claim 10,
wherein the aprotic solvent A comprises at least one ether solvent.

12. The method of producing perfluoro(2-methylene-4-methyl-1,3-dioxolane) according to claims 10 or 11,
wherein the aprotic solvent B comprises at least one alkane solvent.

13. The method of producing perfluoro(2-methylene-4-methyl-1,3-dioxolane) according to claim 12,
wherein the aprotic solvent B comprises liquid paraffin.

14. The method of producing perfluoro(2-methylene-4-methyl-1,3-dioxolane) according to any one of claims 9 to 13,
wherein the raw material solution comprises a perfluoro(2,4-dimethyl-1,3-dioxolane-2-yl)carboxylic acid potassium salt or a perfluoro(2,4-dimethyl-1,3-dioxolane-2-yl)carboxylic acid sodium salt.

15. The method of producing perfluoro(2-methylene-4-methyl-1,3-dioxolane) according to any one of claims 9 to 14, further comprising following processes (1) and (2):
(1) reacting at least one of perfluoro(2,4-dimethyl-2-fluoroformyl-1,3-dioxolane) and a hydrolysis product thereof with a basic aqueous solution containing one or more cations selected from the group consisting of alkali metal ions and alkaline earth metal ions and then separating a liquid containing produced perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts by a liquid separation operation; and
(2) performing one or more water content reduction treatments selected from the group consisting of water evaporation and water adsorption on the liquid containing the obtained perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts to obtain perfluoro(2,4-dimethyl-1,3-dioxolane-2-yl)carboxylic acid alkali metal salts or perfluoro(2,4-dimethyl-1,3-dioxolane-2-yl)carboxylic acid alkaline earth metal salts in a solution state or a solid state.

## Patentansprüche

1. Verfahren zur Herstellung von Perfluor(2-Methylen-4-Methyl-1,3-Dioxolan), wobei das Verfahren mindestens die folgenden Prozesse (1) bis (3) umfasst:
(1) Reagieren von mindestens einem von Perfluor(2,4-Dimethyl-2-Fluorformyl-1,3-Dioxolan) und einem Hydrolyseprodukt davon mit einer basischen wässrigen Lösung, die ein oder mehrere Kationen enthält, die ausgewählt werden aus der Gruppe, die besteht aus Alkalimetallionen und alkalischen Erdmetallionen, und dann Trennen einer Flüssigkeit, die hergestellte Perfluor(2,4-Dimethyl-1,3-Dioxolan-2-yl)-Carbonsäure-Alkalimetallsalze oder alkalische Perfluor(2,4-Dimethyl-1,3-Dioxolan-2-yl)-Carbonsäure-Erdmetallsalze enthält, durch einen Flüssigkeitstrennungsvorgang;
(2) Durchführen von einer oder mehreren Wassergehaltsreduktionsbehandlungen, die ausgewählt werden aus der Gruppe, die besteht aus Wasserverdampfung und Wasseradsorption, auf der Flüssigkeit, welche die erhaltenen Perfluor(2,4-Dimethyl-1,3-Dioxolan-2-yl)-Carbonsäure-Alkalimetallsalze oder alkalischen Perfluor(2,4-Dimethyl-1,3-Dioxolan-2-yl)-Carbonsäure-Erdmetallsalze enthält, um Perfluor(2,4-Dimethyl-1,3-Dioxolan-2-yl)-Carbonsäure-Alkalimetallsalze oder alkalische Perfluor(2,4-Dimethyl-1,3-Dioxolan-2-yl)-Carbonsäure-Erdmetallsalze in einem gelösten Zustand oder in einem festen Zustand zu erhalten; und
(3) Bewirken einer Decarboxylierungsreaktion in einem Flüssigphasensystem mit den erhaltenen Perfluor(2,4-Dimethyl-1,3-Dioxolan-2-yl)-Carbonsäure-Alkalimetallsalzen oder alkalischen Perfluor(2,4-Dimethyl-1,3-Dioxolan-2-yl)-Carbonsäure-Erdmetallsalzen in einem gelösten Zustand oder in einem festen Zustand, um Perfluor(2-Methylen-4-Methyl-1,3-Dioxolan) herzustellen.

2. Verfahren zur Herstellung von Perfluor(2-Methylen-4-Methyl-1,3-Dioxolan) nach Anspruch 1, das ferner einen folgenden Prozess (4) umfasst, nachdem die Flüssigkeit, welche die Perfluor(2,4-Dimethyl-1,3-Dioxolan-2-yl)-Carbonsäure-Alkalimetallsalze oder alkalischen Perfluor(2,4-Dimethyl-1,3-Dioxolan-2-yl)-Carbonsäure-Erdmetallsalze enthält, durch den Flüssigkeitstrennungsvorgang im Prozess (1) getrennt wurde;
(4) mindestens einmaliges Durchführen eines Vorgangs, in dem die Flüssigkeit, welche die Perfluor(2,4-Dimethyl-1,3-Dioxolan-2-yl)-Carbonsäure-Alkalimetallsalze oder alkalischen Perfluor(2,4-Dimethyl-1,3-Dioxolan-2-yl)-Carbonsäure-Erdmetallsalze enthält, die im Prozess (1) erhalten wurde, zusätzlich mit Wasser oder Wasser, in dem ein anorganisches Salz aufgelöst ist, in Kontakt gebracht wird, dann ein Flüssigkeitstrennungsvorgang derart durchgeführt wird, dass die Flüssigkeit, welche die Perfluor(2,4-Dimethyl-1,3-Dioxolan-2-yl)-Carbonsäure-Alkalimetallsalze oder alkalischen Perfluor(2,4-Dimethyl-1,3-Dioxolan-2-yl)-Carbonsäure-Erdmetallsalze enthält, erneut getrennt wird.

3. Verfahren zur Herstellung von Perfluor(2-Methylen-4-Methyl-1,3-Dioxolan) nach Anspruch 1 oder 2, das ferner das Zugeben eines oder mehrerer aprotischer Lösungsmittel C an zumindest einem beliebigen Zeitpunkt von Folgendem umfasst: bevor die basische wässrige Lösung, die ein oder mehrere Kationen enthält, die ausgewählt werden aus der Gruppe, die besteht aus Alkalimetallionen und alkalischen Erdmetallionen, zum Reagieren gebracht wird; nach der Reaktion; und nach dem Flüssigkeitstrennungsvorgang im Prozess (1).

4. Verfahren zur Herstellung von Perfluor(2-Methylen-4-Methyl-1,3-Dioxolan) nach Anspruch 3,
wobei das aprotische Lösungsmittel C ein Etherlösungsmittel ist.

5. Verfahren zur Herstellung von Perfluor(2-Methylen-4-Methyl-1,3-Dioxolan) nach einem der Ansprüche 1 bis 4,
wobei die Wassergehaltsreduktionsbehandlung im Prozess (2) Wasserverdampfung bei Vorhandensein von einem oder mehreren azeotropen Lösungsmitteln umfasst, die eine azeotrope Zusammensetzung mit Wasser bilden.

6. Verfahren zur Herstellung von Perfluor(2-Methylen-4-Methyl-1,3-Dioxolan) nach einem der Ansprüche 3 bis 4,
wobei die Wassergehaltsreduktionsbehandlung im Prozess (2) Wasserverdampfung bei Vorhandensein von einem oder mehreren azeotropen Lösungsmitteln umfasst, die eine azeotrope Zusammensetzung mit Wasser bilden und einen Siedepunkt aufweisen, der niedriger ist als derjenige des aprotischen Lösungsmittels C, das in dem Prozess (1) zugegeben wird.

7. Verfahren zur Herstellung von Perfluor(2-Methylen-4-Methyl-1,3-Dioxolan) nach Anspruch 5 oder 6,
wobei das azeotrope Lösungsmittel eines oder mehrere Lösungsmittel ist, die ausgewählt werden aus der Gruppe, die besteht aus Isopropylalkohol und Toluol.

8. Verfahren zur Herstellung von Perfluor(2-Methylen-4-Methyl-1,3-Dioxolan) nach einem der Ansprüche 1 bis 7,
wobei das Lösungsmittel, das in dem Flüssigphasensystem enthalten ist, wenn die Decarboxylierungsreaktion in dem Flüssigphasensystem im Prozess (3) bewirkt wird, ein Glucoldietherlösungsmittel umfasst.

9. Verfahren zur Herstellung von Perfluor(2-Methylen-4-Methyl-1,3-Dioxolan), wobei das Verfahren einen Reaktivdestillationsprozess umfasst, in dem Reaktivdestillation durch kontinuierliches Zuführen einer Rohstofflösung, welche Perfluor(2,4-Dimethyl-1,3-Dioxolan-2-yl)-Carbonsäure-Alkalimetallsalze oder alkalische Perfluor(2,4-Dimethyl-1,3-Dioxolan-2-yl)-Carbonsäure-Erdmetallsalze enthält, zu einem Reaktivdestillationsapparat durchgeführt wird, um Perfluor(2-Methylen-4-Methyl-1,3-Dioxolan) herzustellen.

10. Verfahren zur Herstellung von Perfluor(2-Methylen-4-Methyl-1,3-Dioxolan) nach Anspruch 9,
wobei der Reaktivdestillationsprozess das kontinuierliche Zuführen einer Rohstofflösung aus in einem aprotischen Lösungsmittel A gelösten Perfluor(2,4-Dimethyl-1,3-Dioxolan-2-yl)-Carbonsäure-Alkalimetallsalzen oder alkalischen Perfluor(2,4-Dimethyl-1,3-Dioxolan-2-yl)-Carbonsäure-Erdmetallsalzen zu einem aprotischen Lösungsmittel B, das in dem Reaktivdestillationsapparat unter einem verminderten Druck erhitzt wird, und Durchführen von Reaktion bei gleichzeitigem Herausdestillieren des hergestellten Perfluor(2-Methylen-4-Methyl-1,3-Dioxolans) aus dem System umfasst, und
das aprotische Lösungsmittel B ein Lösungsmittel ist, das das gleiche wie das aprotische Lösungsmittel A ist oder sich davon unterscheidet und einen Siedepunkt aufweist, der gleich oder höher als derjenige des aprotischen Lösungsmittels A ist.

11. Verfahren zur Herstellung von Perfluor(2-Methylen-4-Methyl-1,3-Dioxolan) nach Anspruch 10,
wobei das aprotische Lösungsmittel A mindestens ein Etherlösungsmittel umfasst.

12. Verfahren zur Herstellung von Perfluor(2-Methylen-4-Methyl-1,3-Dioxolan) nach Anspruch 10 oder 11,
wobei das aprotische Lösungsmittel B mindestens ein Alkanlösungsmittel umfasst.

13. Verfahren zur Herstellung von Perfluor(2-Methylen-4-Methyl-1,3-Dioxolan) nach Anspruch 12,
wobei das aprotische Lösungsmittel B flüssiges Paraffin umfasst.

14. Verfahren zur Herstellung von Perfluor(2-Methylen-4-Methyl-1,3-Dioxolan) nach einem der Ansprüche 9 bis 13,
wobei die Rohstofflösung ein Perfluor(2,4-Dimethyl-1,3-Dioxilan-2-yl)-Carbonsäure-Kalisalz oder ein Perfluor(2,4-Dimethyl-1,3-Dioxilan-2-yl)-Carbonsäure-Natriumsalz umfasst.

15. Verfahren zur Herstellung von Perfluor(2-Methylen-4-Methyl-1,3-Dioxolan) nach einem der Ansprüche 9 bis 14, das ferner die folgenden Prozesse (1) und (2) umfasst:
(1) Reagieren von mindestens einem von Perfluor(2,4-Dimethyl-2-Fluorformyl-1,3-Dioxolan) und einem Hydrolyseprodukt davon mit einer basischen wässrigen Lösung, die ein oder mehrere Kationen enthält, die ausgewählt werden aus der Gruppe, die besteht aus Alkalimetallionen und alkalischen Erdmetallionen, und dann Trennen einer Flüssigkeit, die hergestellte Perfluor(2,4-Dimethyl-1,3-Dioxolan-2-yl)-Carbonsäure-Alkalimetallsalze oder alkalische Perfluor(2,4-Dimethyl-1,3-Dioxolan-2-yl)-Carbonsäure-Erdmetallsalze enthält, durch einen Flüssigkeitstrennungsvorgang; und
(2) Durchführen von einer oder mehreren Wassergehaltsreduktionsbehandlungen, die ausgewählt werden aus der Gruppe, die besteht aus Wasserverdampfung und Wasseradsorption, auf der Flüssigkeit, welche die erhaltenen Perfluor(2,4-Dimethyl-1,3-Dioxolan-2-yl)-Carbonsäure-Alkalimetallsalze oder alkalischen Perfluor(2,4-Dimethyl-1,3-Dioxolan-2-yl)-Carbonsäure-Erdmetallsalze enthält, um Perfluor(2,4-Dimethyl-1,3-Dioxolan-2-yl)-Carbonsäure-Alkalimetallsalze oder alkalische Perfluor(2,4-Dimethyl-1,3-Dioxolan-2-yl)-Carbonsäure-Erdmetallsalze in einem gelösten Zustand oder in einem festen Zustand zu erhalten.

## Revendications

1. Méthode de production de perfluoro(2-méthylène-4-méthyl-1,3-dioxolane), la méthode comprenant au moins les procédés (1) à (3) suivants :
(1) réaction d'au moins l'un parmi le perfluoro(2,4-diméthyl-2-fluoroformyl-1,3-dioxolane) et un produit d'hydrolyse de celui-ci avec une solution aqueuse basique contenant un ou plusieurs cations choisis dans le groupe constitué par les ions de métal alcalin et les ions de métal alcalino-terreux et ensuite séparation d'un liquide contenant les sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique produits par une opération de séparation de liquide ;
(2) mise en œuvre d'un ou plusieurs traitements de réduction de la teneur en eau choisis dans le groupe constitué par une évaporation d'eau et une adsorption d'eau sur le liquide contenant les sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique obtenus pour que soit obtenu des sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique dans un état en solution ou un état solide ; et
(3) incitation d'une réaction de décarboxylation dans un système en phase liquide avec les sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique obtenus dans un état en solution ou un état solide pour produire du perfluoro(2-méthylène-4-méthyl-1,3-dioxolane).

2. Méthode de production de perfluoro(2-méthylène-4-méthyl-1,3-dioxolane) selon la revendication 1, comprenant en outre le procédé (4) qui suit après que le liquide contenant les sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique est séparé par l'opération de séparation de liquide dans le procédé (1) :
(4) mise en œuvre d'une opération au moins une fois, dans laquelle le liquide contenant les sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique dans le procédé (1) est de plus mis en contact avec de l'eau ou de l'eau dans laquelle un sel inorganique est dissous, et une opération de séparation de liquide est ensuite effectuée de façon que le liquide contenant les sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique soit de nouveau séparé.

3. Méthode de production de perfluoro(2-méthylène-4-méthyl-1,3-dioxolane) selon la revendication 1 ou 2, comprenant en outre l'addition d'un ou plusieurs solvants aprotiques C au moins à n'importe quel moment parmi : avant que la solution aqueuse basique contenant un ou plusieurs cations choisis dans le groupe constitué par les ions de métal alcalin et les ions de métal alcalino-terreux soit mise à réagir ; après la réaction ; et après l'opération de séparation de liquide dans le procédé (1).

4. Méthode de production de perfluoro(2-méthylène-4-méthyl-1,3-dioxolane) selon la revendication 3, dans laquelle le solvant aprotique C est un solvant de type éther.

5. Méthode de production de perfluoro(2-méthylène-4-méthyl-1,3-dioxolane) selon l'une quelconque des revendications 1 à 4, dans laquelle le traitement de réduction de la teneur en eau dans le procédé (2) comprend l'évaporation d'eau en présence d'un ou plusieurs solvants azéotropiques pour former une composition azéotropique avec l'eau.

6. Méthode de production de perfluoro(2-méthylène-4-méthyl-1,3-dioxolane) selon l'une quelconque des revendications 3 et 4, dans laquelle le traitement de réduction de la teneur en eau dans le procédé (2) comprend l'évaporation d'eau en présence d'un ou plusieurs solvants azéotropiques qui forment une composition azéotropique avec l'eau et ont un point d'ébullition inférieur à celui du solvant aprotique C qui est ajouté dans le procédé (1).

7. Méthode de production de perfluoro(2-méthylène-4-méthyl-1,3-dioxolane) selon la revendication 5 ou 6, dans laquelle le solvant azéotropique est un ou plusieurs solvants choisis dans le groupe constitué par l'alcool isopropylique et le toluène.

8. Méthode de production de perfluoro(2-méthylène-4-méthyl-1,3-dioxolane) selon l'une quelconque des revendications 1 à 7, dans laquelle le solvant contenu dans le système en phase liquide quand la réaction de décarboxylation est incitée dans le système en phase liquide dans le procédé (3) comprend un solvant de type diéther de glycol.

9. Méthode de production de perfluoro(2-méthylène-4-méthyl-1,3-dioxolane), la méthode comprenant un procédé de distillation réactionnelle dans lequel une distillation réactionnelle est effectuée par fourniture en continu, à un distillateur réactionnel, d'une solution de matières premières contenant des sels de métal alcalin et d'acide perfluoro(2,4-diméthyl-1,3-dioxolan-2-yl)carboxylique ou des sels de métal alcalino-terreux et d'acide perfluoro(2,4-diméthyl-1,3-dioxolan-2-yl)carboxylique pour produire du perfluoro(2-méthylène-4-méthyl-1,3-dioxolane).

10. Méthode de production de perfluoro(2-méthylène-4-méthyl-1,3-dioxolane) selon la revendication 9,
dans laquelle le procédé de distillation réactionnelle comprend la fourniture en continu d'une solution de matières premières contenant des sels de métal alcalin et d'acide perfluoro(2,4-diméthyl-1,3-dioxolan-2-yl)carboxylique ou des sels de métal alcalino-terreux et d'acide perfluoro(2,4-diméthyl-1,3-dioxolan-2-yl)carboxylique dissous dans un solvant aprotique A à un solvant aprotique B chauffé dans le distillateur réactionnel sous une pression réduite, et la mise en oeuvre d'une réaction cependant que le perfluoro(2-méthylène-4-méthyl-1,3-dioxolane) produit est chassé par distillation vers l'extérieur du système, et
le solvant aprotique B est un solvant qui est identique au ou différent du solvant aprotique A et a un point d'ébullition égal ou supérieur à celui du solvant aprotique A.

11. Méthode de production de perfluoro(2-méthylène-4-méthyl-1,3-dioxolane) selon la revendication 10, dans laquelle le solvant aprotique A comprend au moins un solvant de type éther.

12. Méthode de production de perfluoro(2-méthylène-4-méthyl-1,3-dioxolane) selon la revendication 10 ou 11, dans laquelle le solvant aprotique B comprend au moins un solvant de type alcane.

13. Méthode de production de perfluoro(2-méthylène-4-méthyl-1,3-dioxolane) selon la revendication 12, dans laquelle le solvant aprotique B comprend une paraffine liquide.

14. Méthode de production de perfluoro(2-méthylène-4-méthyl-1,3-dioxolane) selon l'une quelconque des revendications 9 à 13, dans laquelle la solution de matières premières comprend un sel potassique d'acide perfluoro(2,4-diméthyl-1,3-dioxolan-2-yl)carboxylique ou un sel sodique d'acide perfluoro(2,4-diméthyl-1,3-dioxolan-2-yl)carboxylique.

15. Méthode de production de perfluoro(2-méthylène-4-méthyl-1,3-dioxolane) selon l'une quelconque des revendications 9 à 14, comprenant en outre les procédés (1) et (2) suivants :
(1) réaction d'au moins l'un parmi le perfluoro(2,4-diméthyl-2-fluoroformyl-1,3-dioxolane) et un produit d'hydrolyse de celui-ci avec une solution aqueuse basique contenant un ou plusieurs cations choisis dans le groupe constitué par les ions de métal alcalin et les ions de métal alcalino-terreux et ensuite séparation d'un liquide contenant les sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique produits par une opération de séparation de liquide ; et
(2) mise en oeuvre d'un ou plusieurs traitements de réduction de la teneur en eau choisis dans le groupe constitué par une évaporation d'eau et une adsorption d'eau sur le liquide contenant les sels de métal alcalin et d'acide perfluoro(2,4-diméthyl-1,3-dioxolan-2-yl) carboxylique ou les sels de métal alcalino-terreux et d'acide perfluoro(2,4-diméthyl-1,3-dioxolan-2-yl) carboxylique obtenus pour que soit obtenu des sels de métal alcalin et d'acide perfluoro(2,4-diméthyl-1,3-dioxolan-2-yl)carboxylique ou les sels de métal alcalino-terreux et d'acide perfluoro(2,4-diméthyl-1,3-dioxolan-2-yl)carboxylique dans un état en solution ou un état solide.
